# EUROPÄISCHE PATENTSCHRIFT

(11) **EP 1 999 113 B1**
(45) Veröffentlichungstag und Bekanntmachung des Hinweises auf die Patenterteilung: **19.08.2009**
(21) Anmeldenummer: 07711844.6
(22) Anmeldetag: 08.03.2007
(51) Int. Cl.: C07D 217/22, C07D 401/10, A61P 11/00, A61K 31/472, A61K 31/4725

(54) **SUBSTITUIERTE 1-AMINO-4-PHENYL-DIHYDROISOCHINOLINE, VERFAHREN ZU IHRER HERSTELLUNG, IHRE VERWENDUNG ALS MEDIKAMENT, SOWIE SIE ENTHALTENDES MEDIKAMENT**
SUBSTITUTED 1-AMINO-4-PHENYL-DIHYDROISOQUINOLINES, METHOD FOR THE PRODUCTION THEREOF, USE THEREOF AS A MEDICAMENT, AND MEDICAMENTS CONTAINING THEM
1-AMINO-4-PHÉNYL-DIHYDROISOQUINOLÉINES SUBSTITUÉES, PROCÉDÉ DE PRÉPARATION, UTILISATION EN TANT QUE MÉDICAMENT ET MÉDICAMENT LES CONTENANT

(30) Priorität: 18.03.2006 DE 102006012544
(43) Veröffentlichungstag der Anmeldung: 10.12.2008
(73) Patentinhaber: Sanofi-Aventis, 75013 Paris (FR)
(72) Erfinder: LANG, Hans-Jochen, 65926 Frankfurt am Main (DE); WESTON, John, 65926 Frankfurt am Main (DE); HEINELT, Uwe, 65926 Frankfurt am Main (DE)
(74) Vertreter: Kujath, Eckard
(86) Internationale Anmeldenummer: PCT/EP2007/001982
(87) Internationale Veröffentlichungsnummer: WO 2007/107245

(56) Entgegenhaltungen:
- WO-A-02/40449
- WO-A-02/40450
- WO-A-03/055880
- WO-A-20/04085404
- WO-A-20/05028444
- DATABASE BEILSTEIN BEILSTEIN INSTITUTE FOR ORGANIC CHEMISTRY, FRANKFURT-MAIN, DE; BRN 9222885 2002, XP002442959 & HIREMATH S P ET AL: INDIAN JOURNAL OF CHEMISTRY SECTION B, Bd. 41, Nr. 2, 2002, Seiten 394-399,

## Beschreibung

Die Erfindung betrifft Verbindungen vom Typ der substituierten 1-Amino-4-phenyl-dihydroisochinoline. Medikamente, die Verbindungen dieses Typs enthalten, sind nützlich bei der Prävention oder Behandlung diverser Erkrankungen. So lassen sich die Verbindungen unter anderem bei Nierenerkrankungen wie akutem oder chronischem Nierenversagen, bei Störungen der Gallenfunktion, bei Atemstörungen wie Schnarchen oder Schlafapnoen oder bei Schlaganfall einsetzen.

Die Erfindung betrifft Verbindungen der Formel I 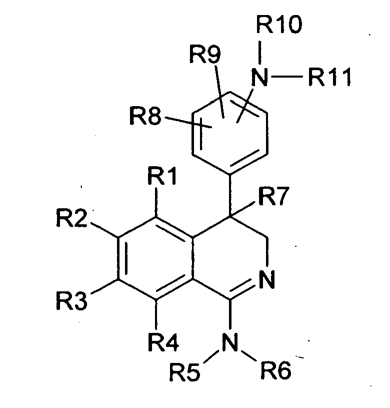 worin bedeuten:
R1, R2, R3 und R4
unabhängig voneinander Wasserstoff, F, Cl, Br, I, CN, NO₂, CF₃, CH₃-SO₂, Alkyl mit 1, 2, 3 oder 4 C-Atomen, NH₂, NH-CH₃ oder N(CH₃)₂;
R5 und R6
unabhängig voneinander Wasserstoff, Alkyl mit 1, 2, 3, 4, 5 oder 6 C-Atomen, CF₃-CH₂-, Cycloalkyl mit 3, 4, 5 oder 6 C-Atomen oder Cyclopropyl-CH₂-,
oder
R5 und R6
bilden gemeinsam mit dem Stickstoffatom, an das sie gebunden sind, einen 4-, 5-, 6-, 7-, 8-, 9- oder 10-gliedrigen Ring, in dem ein oder zwei CH₂-Gruppen unabhängig voneinander durch NR12, Schwefel, Sauerstoff, C(O) oder SO₂ ersetzt sein können;
R12 Wasserstoff, Alkyl mit 1, 2, 3 oder 4 C-Atomen oder Cycloalkyl mit 3, 4, 5 oder 6 C-Atomen;
R7 Wasserstoff oder Alkyl mit 1, 2, 3 oder 4 C-Atomen;
R8 und R9
unabhängig voneinander Wasserstoff, F, Cl, Br, OH, Alkyl mit 1, 2, 3 oder 4 C-Atomen, CH₃O, CF₃, oder CH₃SO₂;
R10 und 11
unabhängig voneinander R13-(CₘH₂ₘ)-Bₙ, wobei
- m: Null, 1, 2, 3 oder 4;
- n: Null oder 1;
- B: -CO-, -CONR14- oder -SO₂-;
R14 Wasserstoff oder Alkyl mit 1, 2, 3, 4, 5 oder 6 C-Atomen;
R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl), 1-Morpholinyl, -COOR15, OR16, NR17R18 oder Phenyl, das unabhängig voneinander 1 oder 2 Substituenten trägt, ausgewählt aus der Gruppe Chlor, Fluor, Methyl und Methoxy;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei, drei oder vier CH₂-Gruppen unabhängig voneinander durch NR19, Schwefel, Sauerstoff, C(O) oder SO₂ ersetzt sein können;
R19 Wasserstoff, Alkyl mit 1, 2, 3 oder 4 C-Atomen oder Cycloalkyl mit 3, 4, 5 oder 6 C-Atomen;
sowie deren pharmazeutisch verträgliche Salze und Trifluoracetate.
Bevorzugt sind Verbindungen der Formel I, worin bedeuten
R1, R2, R3 und R4
unabhängig voneinander Wasserstoff, F, Cl, Br, CN, CF₃, CH₃-SO₂, Alkyl mit 1, 2, 3 oder 4 C-Atomen, NH₂, NH-CH₃ oder N(CH₃)₂;
R5 und R6
unabhängig voneinander Wasserstoff, Alkyl mit 1, 2, 3, 4, 5 oder 6 C-Atomen, CF₃-CH₂-, Cycloalkyl mit 3, 4, 5 oder 6 C-Atomen oder Cyclopropyl-CH₂-,
oder
R5 und R6
bilden gemeinsam mit dem Stickstoffatom, an das sie gebunden sind, einen 4-, 5-, 6-, 7-, 8-, 9- oder 10-gliedrigen Ring;
R7 Wasserstoff oder Methyl;
R8 und R9
unabhängig voneinander Wasserstoff, F, Cl, OH, Alkyl mit 1, 2, 3 oder 4 C-Atomen, CH₃O, CF₃, oder CH₃SO₂;
R10 und 11
unabhängig voneinander R13-(CₘH₂ₘ)-Bₙ, wobei
- m: Null, 1, 2, 3 oder 4;
- n: Null oder 1;
- B: -CO-, -CONR14- oder -SO₂-;
R14 Wasserstoff oder Alkyl mit 1, 2, 3, 4, 5 oder 6 C-Atomen;
R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl),1-Morpholinyl, -COOR15, OR16, NR17R18 oder Phenyl, das unabhängig voneinander 1 oder 2 Substituenten trägt, ausgewählt aus der Gruppe Chlor, Fluor, Methyl und Methoxy; R15, R16, R17 und R18 unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei, drei oder vier CH₂-Gruppen unabhängig voneinander durch NR19, Schwefel, Sauerstoff, C(O) oder SO₂ ersetzt sein können;
R19 Wasserstoff oder Alkyl mit 1, 2, 3 oder 4 C-Atomen;
sowie deren pharmazeutisch verträgliche Salze und Trifluoracetate.

Besonders bevorzugt sind Verbindungen der Formel I, worin bedeuten
R1, R2, R3 und R4
unabhängig voneinander Wasserstoff, F, Cl, Br, CN, CF₃, CH₃-SO₂, Methyl, Ethyl, NH₂, NH-CH₃ oder N(CH₃)₂;
R5 und R6
unabhängig voneinander Wasserstoff, Methyl, Ethyl, Isopropyl, CF₃-CH₂ oder Cycloalkyl mit 3, 4, 5 oder 6 C-Atomen;
oder
R5 und R6
bilden gemeinsam mit dem Stickstoffatom, an das sie gebunden sind, einen 4-, 5-, 6-, 7-, 8-, 9- oder 10-gliedrigen Ring;
R7 Wasserstoff oder Methyl;
R8 und R9
unabhängig voneinander Wasserstoff, Cl oder Methyl;
R10 und 11
unabhängig voneinander R13-(CₘH₂ₘ)-Bₙ, wobei
- m: Null, 1, 2, 3 oder 4;
- n: Null oder 1;
- B: -CO-, -CONR14- oder -SO₂-;
R14 Wasserstoff oder Alkyl mit 1, 2, 3, 4, 5 oder 6 C-Atomen;
R13 Wasserstoff, Methyl, Ethyl, Isopropyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen unabhängig voneinander durch NR19 oder C(O) ersetzt sein können; R19 Wasserstoff oder Methyl;
sowie deren pharmazeutisch verträgliche Salze und Trifluoracetate.

Insbesondere bevorzugt sind Verbindungen der Formel I, worin bedeuten
R1 und R3
Wasserstoff;
R2 und R4
unabhängig voneinander Wasserstoff oder Cl;
R5 und R6
unabhängig voneinander Wasserstoff oder Methyl;
oder
R5 und R6
bilden gemeinsam mit dem Stickstoffatom, an das sie gebunden sind, einen 5- oder 6-gliedrigen Ring;
R7 Wasserstoff;
R8 und R9
unabhängig voneinander Wasserstoff oder Cl;
R10 und 11
unabhängig voneinander R13-(CₘH₂ₘ)-Bₙ, wobei
- m: Null, 1, 2, 3 oder 4;
- n: Null oder 1;
- B: -CO- oder -CONR14-;
R14 Wasserstoff oder Methyl;
R13 Wasserstoff, Methyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen unabhängig voneinander durch NR19, oder C(O) ersetzt sein können; R19 Wasserstoff oder Methyl;
sowie deren pharmazeutisch verträgliche Salze und Trifluoracetate.

Sehr bevorzugt sind Verbindungen der Formel I, worin bedeuten
R1 und R3
Wasserstoff;
R2 und R4
unabhängig voneinander Wasserstoff oder Cl;
R5 und R6
unabhängig voneinander Wasserstoff oder Methyl;
oder
R5 und R6
bilden gemeinsam mit dem Stickstoffatom, an das sie gebunden sind, einen 5-oder 6-gliedrigen Ring;
R7 Wasserstoff;
R8 und R9
unabhängig voneinander Wasserstoff oder Cl;
R10 und 11
unabhängig voneinander R13-(CₘH₂ₘ)-Bₙ, wobei
- m: Null, 1, 2, 3 oder 4;
- n: Null oder 1;
- B: -CONR14-;
R14 Wasserstoff oder Methyl;
R13 Wasserstoff, Methyl oder COOR15;
R15 Wasserstoff, Methyl oder Ethyl;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen unabhängig voneinander durch NR19 oder C(O) ersetzt sein können; R19 Wasserstoff oder Methyl;
sowie deren pharmazeutisch verträgliche Salze und Trifluoracetate.

Speziell bevorzugt sind Verbindungen der Formel I ausgewählt aus der Gruppe:
1-Amino-4-(4-aminophenyl)-6-chlor-3,4-dihydroisochinolin,
1-Amino-4-(4-aminophenyl)- 3,4-dihydroisochinolin,
1-Amino-4-(2-aminophenyl)-6-chlor-3,4-dihydroisochinolin,
4-(4-Aminophenyl)-6-chlor-1-methylamino-3,4-dihydroisochinolin,
4-(4-Aminophenyl)-6-chlor-1-dimethylamino-3,4-dihydroisochinolin,
4-(4-Aminophenyl)-6-chlor-1-(N-pyrrolidino)-3,4-dihydroisochinolin,
N-(Ethoxycarbonymethyl)-N'-4-[(6-chloro-3,4-dihydro-1-dimethylaminoisochinolin-4-yl)phenyl]harnstoff
   und
3-{4-[(6-Chlor-3,4-dihydro-1-dimethylaminoisochinolin-4-yl)phenyl]}- imidazolidin-2,4-dion
sowie deren pharmazeutisch verträgliche Salze und Trifluoracetate.

In einer Ausführungsform sind Verbindungen der Formel I bevorzugt, in denen die Reste R1, R2, R3 und R4 unabhängig voneinander beschrieben werden durch Wasserstoff, F, Cl, Br, CN, CF₃, CH₃-SO₂, Alkyl mit 1, 2, 3 oder 4 C-Atomen, beispielsweise Methyl oder Ethyl, NH₂, NH-CH₃ oder N(CH₃)₂; besonders bevorzugt sind Verbindungen der Formel I, in denen R1 und R3 Wasserstoff sind und R2 und R4 unabhängig voneinander Wasserstoff, F, Cl, Br, CN, CF₃, CH₃-SO₂, Methyl, Ethyl, NH₂, NH-CH₃ oder N(CH₃)₂, beispielsweise Wasserstoff oder Cl, sind; in einer weiteren Ausführungsform sind Verbindungen der Formel I bevorzugt, in denen R1, R3 und R4 Wasserstoff sind und R2 F, Cl, Br, CN, CF₃, CH₃-SO₂, Methyl, Ethyl, NH₂, NH-CH₃ oder N(CH₃)₂, beispielsweise Cl, ist.

In einer Ausführungsform sind Verbindungen der Formel I bevorzugt, in denen R5 und R6 unabhängig voneinander durch Wasserstoff, Alkyl mit 1, 2, 3, 4, 5 oder 6 C-Atomen, CF₃-CH₂- oder Cycloalkyl mit 3, 4, 5 oder 6 C-Atomen beschrieben werden oder R5 und R6 gemeinsam mit dem Stickstoffatom, an das sie gebunden sind, einen 4-, 5-, 6-, 7-, 8-, 9- oder 10-gliedrigen Ring bilden; in einer weiteren Ausführungsform sind Verbindungen der Formel I bevorzugt, in denen R5 und R6 unabhängig voneinander Wasserstoff, Methyl, Ethyl, Isopropyl, CF₃-CH₂ oder Cycloalkyl mit 3, 4, 5 oder 6 C-Atomen sind oder R5 und R6 gemeinsam mit dem Stickstoffatom, an das sie gebunden sind, einen 4-, 5-, 6-, 7-, 8-, 9- oder 10-gliedrigen Ring bilden; besonders bevorzugt sind Verbindungen der Formel I, in denen R5 und R6 Wasserstoff, Methyl oder Ethyl, beispielsweise Wasserstoff oder Methyl, bedeuten oder R5 und R6 gemeinsam mit dem Stickstoffatom, an das sie gebunden sind, einen 5- oder 6- gliedrigen Ring bilden, insbesondere Pyrrolidin oder Piperidin, beispielsweise Pyrrolidin.

In einer weiteren Ausführungsform sind Verbindungen der Formel I bevorzugt, in denen R7 durch Wasserstoff oder Methyl, beispielsweise Wasserstoff, beschrieben wird.

In einer Ausführungsform sind Verbindungen der Formel I bevorzugt, in denen die Reste R8 und R9 unabhängig voneinander beschrieben werden durch Wasserstoff, F, Cl, OH, Alkyl mit 1, 2, 3 oder 4 C-Atomen, CH₃O, CF₃, oder CH₃SO₂; besonders bevorzugt sind Verbindungen der Formel I, in denen R8 und R9 unabhängig voneinander beschrieben werden durch Wasserstoff, Cl oder Methyl, insbesondere Wasserstoff oder Cl, beispielsweise Wasserstoff.

Der Rest NR10R11 am Phenylring kann in ortho-, meta- oder para-Position zur Dihydroisochinolingruppe gebunden sein, beispielsweise in ortho- oder para-Position, insbesondere in para-Position.

In einer Ausführungsform sind Verbindungen der Formel I bevorzugt, in denen die Reste R10 und R11 unabhängig voneinander beschrieben werden durch R13-(CₘH₂ₘ)-Bₙ oder R10 und R11 bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen durch NR19 oder C(O) ersetzt sein können, wobei R19 Wasserstoff oder Alkyl mit 1, 2, 3 oder 4 C-Atomen, insbesondere Wasserstoff oder Methyl, ist; in einer weiteren Ausführungsform sind Verbindungen der Formel I bevorzugt, in denen die Reste R10 und R11 unabhängig voneinander beschrieben werden durch R13-(CₘH₂ₘ)-Bₙ. In einer weiteren Ausführungsform werden Verbindungen der Formel I bevorzugt, in denen R10 und R11 gemeinsam mit dem Stickstoffatom, an das sie gebunden sind, einen 4-, 5-, 6-, 7-, 8-, 9- oder 10-gliedrigen Ring bilden, in dem ein, zwei oder drei CH₂-Gruppen durch NR19 oder C(O) ersetzt sein können, beispielsweise sind 3 CH₂-Gruppen durch ein NR19 und zwei C(O) ersetzt, wobei R19 Wasserstoff oder Methyl, ist;

In einer Ausführungsform sind Verbindungen der Formel I bevorzugt, in denen m Null, 1 oder 2, beispielsweise Null oder 1, ist.

In einer Ausführungsform sind Verbindungen der Formel I bevorzugt, in denen n Null ist; in einer weiteren Ausführungsform sind Verbindungen der Formel I bevorzugt, in denen n 1 ist.

In einer Ausführungsform sind Verbindungen der Formel I bevorzugt, in denen B -CO-oder -CONR14-, insbesondere -CONR14, ist, wobei R14 Wasserstoff oder Alkyl mit 1, 2, 3, 4, 5 oder 6 C-Atomen, insbesondere Wasserstoff oder Methyl, beispielsweise Wasserstoff, ist.

In einer Ausführungsform sind Verbindungen der Formel I bevorzugt, in denen R13 beschrieben wird durch Wasserstoff, Methyl, Ethyl, Isopropyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18, wobei R15, R16, R17 und R18 unabhängig,voneinander Wasserstoff oder Alkyl mit 1, 2, 3, 4, 5 oder 6 C-Atomen sind; in einer weiteren Ausführungsform sind Verbindungen der Formel I bevorzugt, in denen R13 beschrieben wird durch Wasserstoff, Methyl oder -COOR15, wobei R15 Wasserstoff oder Alkyl mit 1, 2, 3, 4, 5 oder 6 C-Atomen, insbesondere Wasserstoff, Methyl oder Ethyl, beispielsweise Ethyl, ist; besonders bevorzugt sind Verbindungen der Formel I, in denen R13 Wasserstoff oder-COOR15 ist, wobei R15 Ethyl ist.

Enthalten die Verbindungen der Formel I ein oder mehrere Asymmetriezentren, so können diese unabhängig voneinander sowohl S als auch R konfiguriert sein. Die Verbindungen können als optische Isomere, als Diastereomere, als Racemate oder als Gemische in allen Verhältnissen derselben vorliegen. Die Verbindungen der Formel I können weiterhin in Form von Rotationsisomeren vorliegen.

Die vorliegende Erfindung umfasst alle tautomeren Formen der Verbindungen der Formel I.

Die vorliegende Erfindung umfasst weiterhin Derivate der Verbindungen der Formel I, zum Beispiel Solvate, wie Hydrate und Alkoholaddukte, Ester, Prodrugs und andere physiologisch akzeptablen Derivate der Verbindungen der Formel I, sowie aktive Metaboliten der Verbindungen der Formel I. Die Erfindung umfasst ebenfalls alle Kristallmodifikationen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl oder Hexyl. Bevorzugte Alkylreste sind Methyl, Ethyl, n-Propyl, Isopropyl, n-Butyl, tert-Butyl, n-Pentyl, n-Hexyl.

Beispiele für Cycloalkylreste sind Cyclopropyl, Cyclobutyl, Cyclopentyl, Cyclohexyl, Cycloheptyl oder Cyclooctyl. In den Cycloalkylresten können eine oder mehrere CH₂-Gruppen durch O, NH oder N-Alkyl, beispielsweise NCH₃, ersetzt sein. Dies gilt auch für Cycloalkylmethylreste.

Beispiele für Ringe aus NR5R6 sind Morpholin, Pyrrolidin, Piperidin, Piperazin, N-Methylpiperazin, Pyrrolidin-2-on, Pyrrolidin-2,5-dion, Imidazolidin, 3-Methylimidazolidin, Imidazolidin-2-on, 3-Methyl-lmidazolidin-2-on, Imidazolidin-2,4-dion und 1-Methyl-imidazolidine-2,4-dion, insbesondere Pyrrolidin und Piperidin, beispielsweise Pyrrolidin.

Beispiele für Ringe aus NR10R11 sind Morpholin, Pyrrolidin, Piperidin, Piperazin, N-Methylpiperazin, Pyrrolidin-2-on, Pyrrolidin-2,5-dion, Imidazolidin, 3-Methylimidazolidin, Imidazolidin-2-on, 3-Methyl-Imidazolidin-2-on, Imidazolidin-2,4-dion und 1-Methyl-imidazolidine-2,4-dion, insbesondere Pyrrolidin-2,5-dion und Imidazolidin-2,4-dion, beispielsweise Imidazolidin-2,4-dion.

Als CH₂-Einheiten gelten auch die in einem Alkylrest terminalen CH₃-Gruppen, die in diesem Zusammenhang als CH₂-H Gruppierungen aufgefasst werden.

Wenn eine Variable, beispielsweise cycloalkyl oder R1, mehr als einmal als Komponente vorkommt, sind die Definitionen der Variable bei jedem Auftreten unabhängig voneinander.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I an einer sauren Gruppe deprotoniert werden und beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren, verwendet werden. Da Verbindungen der Formel I stets wenigstens eine basische Gruppe enthalten, können sie auch in Form ihrer physiologisch verträglichen Säureadditionssalze z. B. mit folgenden Säuren hergestellt werden: aus anorganischen Säuren wie Salzsäure, Schwefelsäure oder Phosphorsäure oder aus organischen Säuren wie Essigsäure, Zitronensäure, Weinsäure, Milchsäure, Malonsäure, Methansulfonsäure, Fumarsäure. Als Säureadditionssalze kommen dabei Salze aller pharmakologisch verträglichen Säuren in Frage (diese Gruppe entspricht auch den physiologisch akzeptablen Anionen), beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate; aber auch Trifluoracetate.

Gegenstand der Erfindung ist auch das nachfolgend beschriebene Verfahren zur Herstellung der Verbindungen der Formel I.

Die hier beschriebenen Verbindungen der Formel I, in denen R10 und R11 Wasserstoff sind, lassen sich beispielsweise ausgehend von Nitrophenyl-Derivaten der Formel II durch Reduktion zu den entsprechenden Aminoverbindungen der Formel Ia herstellen, 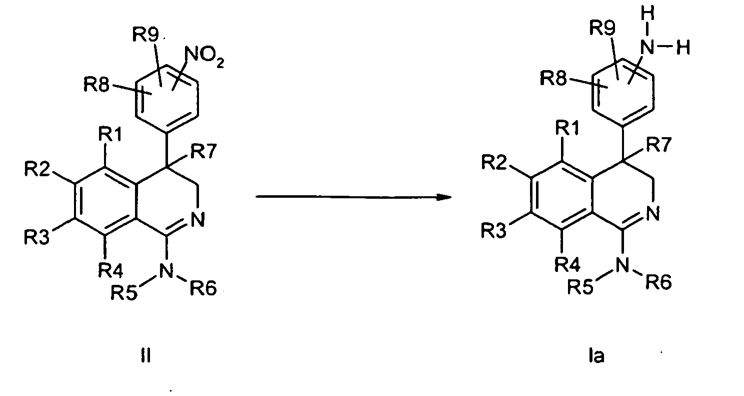 wobei die Substituenten R1, R2, R3, R4, R5, R6, R8 und R9 wie oben beschrieben definiert sind. Die Reduktion kann nach dem Fachmann bekannten Verfahren mit einer Vielzahl von Reduktionsmitteln, einschließlich der katalytischen Hydrierung, erfolgen, beispielsweise durch katalytische Hydrierung oder mit einem anorganischen Reduktionsmittel, wie zum Beispiel mit Eisenpulver und Salzsäure in Eisessig.

Aus den erfindungsgemäßen Verbindungen der Formel I a) lassen sich weitere erfindungsgemäßen Verbindungen der Formel I beispielweise durch Derivatisierung der Aminogruppe am Phenylrest nach dem Fachmann bekannten Verfahren darstellen. Dabei wird beispielsweise die Aminogruppe der Verbindungen Ia) mit Alkylierungsmitteln, Acylierungsmitteln bzw. Sulfonylierungs-Reagentien der Formel R10-L und/oder R11-L vorteilhaft in Gegenwart einer Hilfsbase, wie Pyridin, Triethylamin oder Hünigscher Base, in dem Fachmann bekannter Weise umgesetzt. Ebenfalls eignet sich die Verwendung von Isocyanaten der Formeln R10-N=C=O und/oder R11-N=C=O in dem Fachmann bekannter Weise zur Herstellung entsprechender Harnstoffderivate der Formel Ib oder Ic. 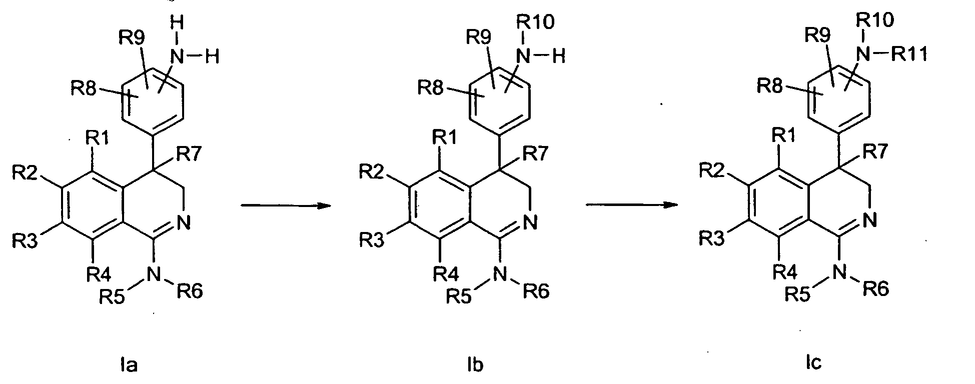 wobei die Substituenten R1, R2, R3, R4, R5, R6, R8, R9, R10 und R11 die oben angegebene Bedeutung haben, R10 und R11 jedoch nicht Wasserstoff sind, und L F, Cl, Br, I, -OR, -OC(O)R oder -SR bedeutet, wobei R Alkyl mit 1, 2, 3, 4, 5 oder 6 C-Atomen, beispielsweise Methyl oder Ethyl, bedeutet und L dann -SR bedeuten kann, wenn B -C(O)- ist. Durch stufenweise erfolgende Umsetzungen kann beispielweise die monosubstituierte erfindungsgemäße Verbindung der Formel Ib erhalten und isoliert werden und/oder anschließend zur disubstituierten Verbindung der Formel Ic umgesetzt werden.

Die Verbindungen der Formel IV können in vielfältiger Weise, beispielsweise durch nucleophilen Austausch mit Verbindungen der Formel III durch dem Fachmann bekannte Verfahren in Verbindungen der Formel II überführt werden, 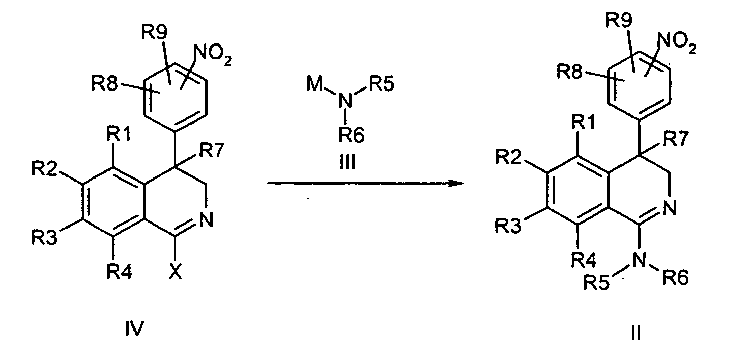 wobei R1, R2, R3, R4, R5, R6, R8, R9 und Y die oben angegebene Bedeutung besitzen, R5 und R6 jedoch nicht beide Wasserstoff bedeuten, X eine nucleophil substituierbare Fluchtgruppe repräsentiert, wie beispielsweise Chlorid, Bromid, Tosylat, Mesylat, Triflat, Alkoxy mit 1, 2, 3, 4, 5 oder 6 C-Atomen, zum Beispiel Ethoxy, Aryloxy, zum Beispiel Phenoxy, oder R'S(O)ₙ-, wobei n 0 oder 2 bedeutet und R' ein Alkylrest, vorzugsweise mit 1, 2, 3 oder 4 C-Atomen, beispielsweise Methyl, ist und M entweder Wasserstoff oder ein Metall, insbesondere ein Alkalimetall oder ein Erdalkalimetalläquivalent bedeutet, beispielsweise Lithium, oder die Verbindung VII eine Grignardverbindung ist. Vorteilhaft arbeitet man mit Aminen unter Anwendung von Temperaturen >100°C, wobei die Verwendung eines Autoklaven oder einer Microwelle empfohlen werden kann.
Entsprechende Verbindungen der Formel IVb, in denen R5 und R6 Wasserstoff bedeuten, können analog durch Umsetzung mit NH₃ und unter Druck hergestellt werden.

Die Verbindungen der Formel IVa mit X = R'-S- können nach dem Fachmann bekannten Verfahren durch Reaktion der entsprechenden Mercapto-Verbindungen der Formel V mit einem Alkylierungsmittel der Formel VI erhalten werden, 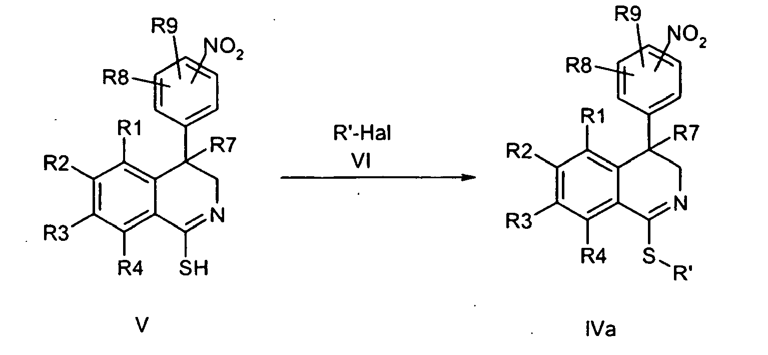 wobei R1, R2, R3, R4, R7, R8 und R9 die oben angegebene Bedeutung besitzen, R' ein Alkylrest, vorzugsweise mit 1, 2, 3 oder 4 C-Atomen, beispielsweise Methyl, und Hal für Chlor, Brom oder Jod steht.

Die Darstellung der Verbindungen der Formel V kann beispielsweise durch säurekatalysierte Cyclisierung der entsprechenden Isothiocyanate der Formel VII nach dem Fachmann bekannten Verfahren erfolgen, 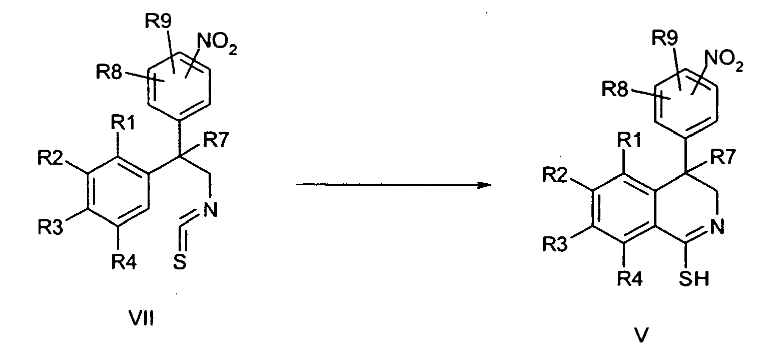 wobei die Substituenten R1, R2, R3, R4, R7, R8 und R9 die oben angegebene Bedeutung besitzen. Als Säuren können sowohl protische wie auch aprotische LewisSäuren verwendet werden. Als vorteilhaft hat sich bei der vorliegenden Cyclisierung die Verwendung konzentrierter Schwefelsäure gezeigt.

Die Isothiocyanate der Formel VII können nach einem dem Fachmann bekannten Verfahren aus den entsprechenden Aminen der Formel VIII mit einem. Thiocarbonylierungsmittel, beispielsweise Thiophosgen S=CCl₂ oder Thiocarbonyl-bisimidazol, hergestellt werden, 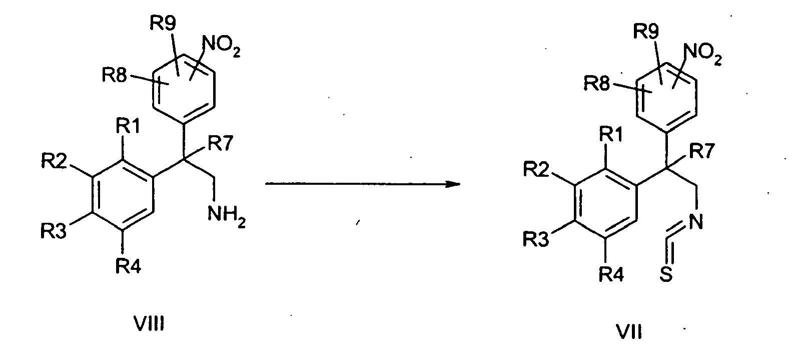 wobei die Substituenten R1, R2, R3, R4, R7, R8 und R9 die oben angegebene Bedeutung besitzen.

Die Phenethylamin-Vorläufer der Formel VIII können, falls nicht käuflich erhältlich, nach dem Fachmann bekannten Standardverfahren beispielsweise aus den entsprechenden Nitrilen der Formel IX durch Reduktion erhalten werden, 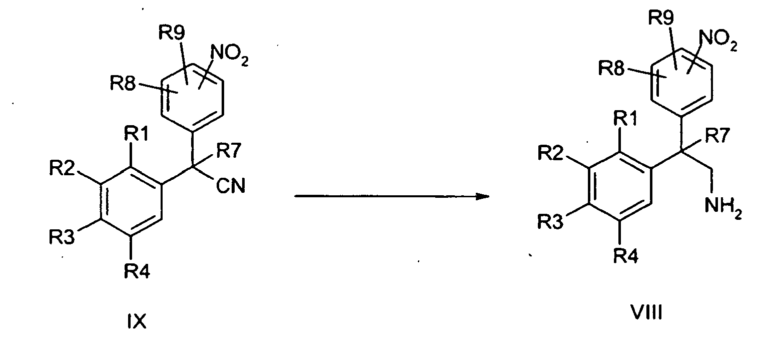 wobei die Substituenten R1, R2, R3, R4, R7, R8 und R9 die oben angegebene Bedeutung besitzen. Bei dieser Reduktion erwies sich die Verwendung von Borwasserstoff-Komplexen als günstig, da sie die Nitrogruppe unberührt lässt, die dann später separat und selektiv, beispielsweise mit Eisenpulver, zur Anilinogruppe reduziert werden kann (Umino, Tetrahedron Letters 33: 2875-76 (1976), J.Org.Chem. 53, 98-104 (1988), Org. Prep. Proced.Int. 13:225 (1981), J.Org.Chem. 47:1389 (1982), Chem.Rev. 76:773 (1973)).

Die Verbindungen der Formel IX, sofern diese nicht käuflich sind, können nach verschiedenen, dem Fachmann bekannten Verfahren hergestellt werden. So kann man beispielsweise von den Verbindungen der Formel XI ausgehen, und diese in dem Fachmann bekannter Weise mittels einer Base durch Erzeugung des Anions der Formel X in einer nucleophilen Substitutionsreaktion am Aromaten mit einer Verbindung der Formel XII umsetzen, 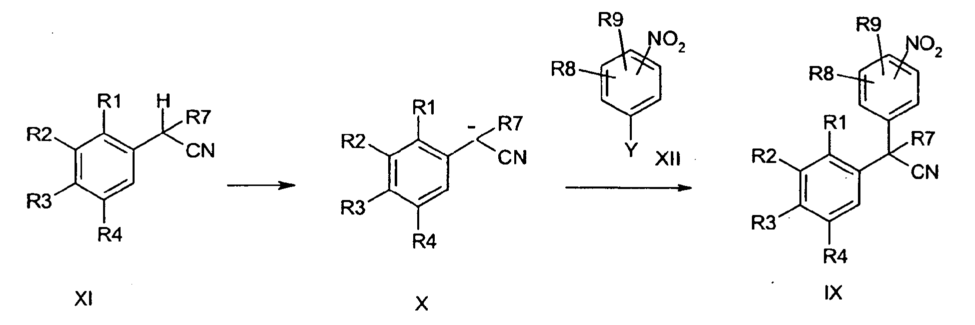 wobei die Substituenten R1, R2, R3, R4, R7, R8 und R9 die oben angegebene Bedeutung besitzen, Y ein nucleophil austauschbarer Rest, beispielsweise Chlorid, Fluorid, Bromid, NO₂, Triflate oder Mesylat ist, und bei der Umsetzung der Verbindung der Formel XI zur Verbindung der Formel X eine beliebige Base, beispielsweise HO⁻, CH₃-O- oder tert.But-O⁻, LDA, NaNH₂ oder K(N(SiMe₃)₂ verwendet wird. Bei dieser Reaktion hat sich normale Alkalilauge unter den Bedingungen der Zweiphasenkatalyse als vorteilhaft erwiesen, wobei als Zweiphasenkatalysator beispielweise N-Benzyl-N,N,N-triethylammonium-chlorid verwendet wird (DE2610837).

Eine Einführung eines Restes R7, der nicht Wasserstoff ist, kann nach dem Fachmann bekannten Verfahren auch auf einer späteren Stufe erfolgen. Beispielsweise kann die Verbindung der Formel IXa mit einer hinreichend starken Base deprotoniert und anschließend mit einem Alkylierungsmittel der Formel XIII umgesetzt werden, 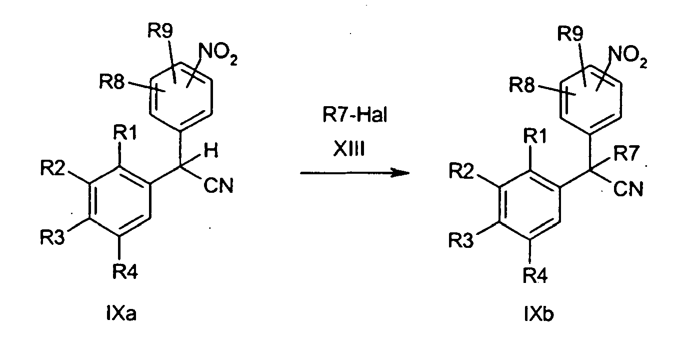 wobei die Substitutenten R1, R2, R3, R4, R7, R8 und R9 und Hal die oben angegebene Bedeutung besitzen.

Auch die Einführung der Nitrogruppe kann in bekannter Weise auf einer späteren Stufe, beispielsweise durch direkte Nitrierungsreaktion einer Verbindung der Formel XIV mit Salpetersäure oder einem Generator für Nitronium, wie beispielsweise [NO₂⁺SbF₆⁻], erfolgen 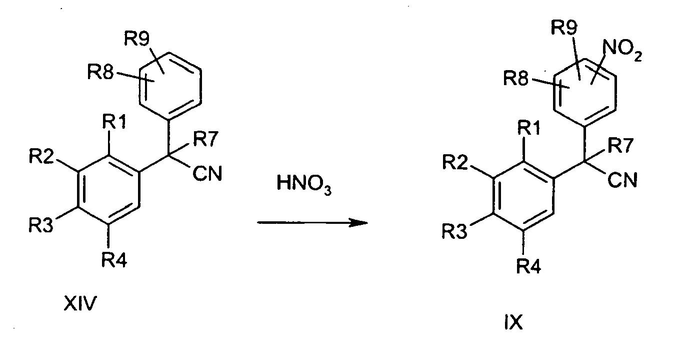 wobei die Substituenten R1, R2, R3, R4, R7, R8 und R9 die oben angegebene Bedeutung besitzen.

Die Verbindungen R10-Hal, R11-Hal, R10-N=C=O, R11-N=C=O und die Verbindungen der Formeln III, VI, XI, XII, XIII und XIV sind käuflich erhältlich oder können nach oder analog zu in der Literatur beschriebenen, dem Fachmann bekannten Verfahren hergestellt werden. Die Verbindungen der Formel IV bzw. IVa sind käuflich erhältlich, können nach den oben beschriebenen Verfahren hergestellt werden (für X = Alkyl-S-) oder können nach oder analog zu in der Literatur beschriebenen, dem Fachmann bekannten Verfahren hergestellt werden. Die Verbindungen der Formeln VIII, IX und IXa und sind käuflich erhältlich, können nach den oben beschriebenen Verfahren hergestellt werden oder können nach oder analog zu in der Literatur beschriebenen, dem Fachmann bekannten Verfahren hergestellt werden.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Es konnte gezeigt werden, dass Verbindungen der Formel I hervorragende Inhibitoren des Natrium-Wasserstoffaustauschers (NHE), insbesondere des Natrium-Wasserstoffaustauschers vom Subtyp 3 (NHE3), darstellen.

Die bisher bekannten NHE3-Inhibitoren leiten sich beispielsweise von Verbindungen des Acylguanidin-Typs (EP825178), Norbornylamin-Typs (W00144164), 2-Guanidinochinazolin-Typs (WO0179186) oder Benzamidin-Typs (WO0121582, W00172742) ab. Das ebenfalls als NHE3-Inhibitor beschriebene Squalamin (M. Donowitz et al. Am. J. Physiol. 276 (Cell Physiol. 45): C136 - C144) wirkt nach derzeitigem Wissensstand nicht unmittelbar wie die Verbindungen nach Formel I, sondern über einen indirekten Mechanismus und erreicht so seine maximale Wirkstärke erst nach einer Stunde.

Tetrahydroisochinoline als Inhibitoren des Natrium-Wasserstoffaustauschers vom Subtyp 3 (NHE3) werden beispielsweise in den Patentanmeldungen W003048129, W02004085404 und den deutschen Anmeldungen 102004046492.8 und 102005001411.9 beschrieben. In der Patentanmeldung WO03055880 ist die verwandte Verbindungsklasse der Tetrahydroisochinolinium-Salze als NHE3-Inhibitoren beschrieben.

Überraschend wurde nun gefunden, dass die hier beschriebenen Verbindungen der Formel I ebenfalls potente Inhibitoren des NHE3 darstellen und dabei vorteilhafte pharmakologische und pharmakokinetische Eigenschaften besitzen.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund ihrer NHE-inhibitorischen Eigenschaften eignen sich die Verbindungen der Formel I zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.
Die Verbindungen der Formel I können auch zur Behandlung und Prävention von Krankheiten eingesetzt werden, wobei der NHE nur partiell gehemmt wird, beispielsweise durch Verwendung einer niedrigeren Dosierung.

Die Verwendung der erfindungsgemäßen Verbindungen betrifft die Prävention und die Behandlung akuter und chronischer Krankheiten in der Veterinär- und in der Humanmedizin.

Infolge Ihrer pharmakologische Wirkungen sind die Verbindungen der Formel I insbesondere dazu geeignet, zu einer Verbesserung des Atemantriebes führen. Sie können deshalb zur Behandlung von gestörten Atmungszuständen herangezogen werden, wie sie beispielsweise bei folgenden klinischen Zuständen und Krankheiten auftreten können: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird. Die genannten Verbindungen finden deshalb vorteilhaft Verwendung zur Herstellung eines Medikaments zur Prävention und Behandlung von Schlafapnoen und muskulär bedingter Atemstörungen und zur Herstellung eines Medikaments zur Prävention und Behandlung des Schnarchens.

Eine Kombination eines NHE-Inhibitors der Formel I mit einem Carboanhydrase-Hemmer (z. B. Acetazolamid) kann sich als vorteilhaft erweisen, wobei letzterer eine metabolische Azidose herbeiführt und dadurch bereits die Atmungstätigkeit steigert, so dass eine verstärkte Wirkung und verminderter Wirkstoffeinsatz erzielt werden können.

Die erfindungsgemäßen Verbindungen schonen infolge ihrer NHE3-inhibitorischen Wirkung die zellulären Energiereserven, die sich unter toxischen und pathogenen Ereignissen rasch erschöpfen und so zur Zellschädigung oder zum Zelluntergang führen. Dabei wird die energieaufwendige ATP-verbrauchende Natriumresorption im proximalen Tubulus unter dem Einfluß von NHE3-Inhibitoren vorübergehend still gelegt und die Zelle kann so eine akute pathogene, ischämische oder toxische Situation überleben. Die Verbindungen eignen sich deshalb beispielsweise als Arzneimittel zur Behandlung ischämischer Noxen, zum Beispiel des akuten Nierenversagens. Weiterhin eignen sich die Verbindungen auch zur Behandlung aller chronischen Nierenerkrankungen und Nephritisformen, die infolge vermehrter Proteinausscheidung zum chronischen Nierenversagen führen. Dementsprechend eignen sich die Verbindungen der Formel I zur Herstellung eines Medikaments zur Behandlung diabetischer Spätschäden, der diabetischen Nephropathie und chronischer Nierenerkrankungen, insbesondere von allen Nierenentzündungen (Nephritiden), die mit einer vermehrten Protein-/ Albuminausscheidung verbunden sind.

Es hat sich gezeigt, dass die erfindungsgemäß verwendeten Verbindungen eine milde abführende Wirkung besitzen und demzufolge auch vorteilhaft als Abführmittel oder bei drohender Darmverstopfung verwendet werden können.

Weiterhin können die erfindungsgemäßen Verbindungen vorteilhaft zur Prävention und Therapie von akuten und chronischen Erkrankungen des Darmtrakts verwendet werden, die beispielweise durch ischämische Zustände im Intestinalbereich und / oder durch nachfolgende Reperfusion oder durch entzündliche Zustände und Ereignisse ausgelöst werden. Solche Komplikationen können beispielweise durch mangelnde Darmperistaltik auftreten, wie sie z.B. häufig nach chirurgischen Eingriffen, bei Darmverstopfung oder stark verminderter Darmtätigkeit zu beobachten sind.

Mit den erfindungsgemäßen Verbindungen besteht die Möglichkeit, der Gallenstein-Bildung vorzubeugen.

Die erfindungsgemäßen NHE-Inhibitoren eignen sich generell zur Behandlung von Krankheiten, die durch Ischämie und durch Reperfusion hervorgerufen werden.

Die erfindungsgemäßen Verbindungen sind infolge ihrer pharmakologischen Eigenschaften als antiarrhythmische Arzneimittel geeignet. Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die erfindungsgemäßen Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den mit Verbindungen der Formel I vorbehandelten Empfängerorganismus verwendet werden können.

Die Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden können die erfindungsgemäßen Verbindungen der Formel I auch bei Wiederbelebung nach einem Herzstillstand eingesetzt werden.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind.

Da NHE-Inhibitoren menschliches Gewebe und Organe nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist deren kombinierte Verabreichung mit Verbindungen der Formel geeignet, die cytotoxischen Effekte einer Therapie zu vermindern oder zu unterdrücken. Durch die Verminderung der cytotoxischen Effekte, insbesondere der Cardiotoxizität, infolge Ko-Medikation mit NHE-Inhibitoren kann außerdem die Dosis der cytotoxischen Therapeutika erhöht und / oder die Medikation mit solchen Arzneimitteln verlängert werden. Der therapeutische Nutzen einer solchen cytotoxischen Therapie kann durch die Kombination mit NHE-Inhibitoren erheblich gesteigert werden.
Die Verbindungen der Formel I eignen sich insbesondere zur Verbesserung der Therapie mit Arzneimitteln, die eine unerwünschte cardiotoxische Komponente besitzen.

Generell können die hier beschriebenen NHE Inhibitoren in günstiger Weise mit anderen, den intrazellulären pH-Wert ebenfalls regulierenden Verbindungen kombiniert werden, wobei Inhibitoren der Enzymgruppe der Carboanhydratasen, Inhibitoren der Bicarbonationen transportierenden Systeme wie des Natrium-Bicarbonat-Cotransporters (NBC) oder des Natrium-abhängigen Chlorid-Bicarbonat-Austauschers (NCBE), sowie mit anderen NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind.

Die Verbindungen der Formel I eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychischer Depressionszustände, Angsterkrankungen und Psychosen. Dabei können die erfindungsgemäßen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Die Verbindungen der Formel I können ebenfalls zur Prävention und zur Behandlung thrombotischer Erkrankungen verwendet werden, da sie als NHE-Inhibitoren sowohl die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und thrombogener Selectin-Proteine, hemmen bzw. verhindern. Damit kann die pathogene Wirkung thrombogener und entzündungsrelevanter Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-RezeptorAntagonisten, Phosphodiesterase-Inhibitoren, Faktor-VIIa-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydratase, wie beispielsweise mit Acetazolamid, ist besonders günstig.

Darüber hinaus zeichnen sich die erfindungsgemäßen NHE-Inhibitoren durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden. Sie können somit zur Behandlung von Organhypertrophien und -hyperplasien, beispielsweise des Herzens und der Prostata verwendet werden. Verbindungen der Formel I sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

NHE-Inhibitoren zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.

Da der NHE bei essentiellen Hypertonikern signifikant erhöht ist, eignen sich die Verbindungen der Formel I zur Prävention und zur Behandlung des Bluthochdrucks und von Herz-Kreislauferkrankungen. Dabei können sie allein oder mit einem geeigneten Kombinationspartner zur Bluthochdruckbehandlung und zur Behandlung von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, mit Verbindungen der Formel I kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromokalim, Minoxidil und deren Derivate, Aktivatoren des mitochondrialen ATP-sensitiven Kaliumkanals (mitoK(ATP) Kanal), Inhibitoren weiterer Kaliumkanäle, wie des Kv1.5 usw.
Infolge ihrer antiphlogistischen Wirkung können erfindungsgemäße NHE-Inhibitoren als Antünflammatorika verwendet werden. Mechanistisch fällt dabei die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antünflammatorika verwendet.

Es wurde außerdem gefunden, dass NHE-Inhibitoren eine günstige Beeinflussung der Serumlipoproteine zeigen. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades. NHE-Inhibitoren der Formel I finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusiansschäden, zur Herstellung eines Medikaments zur Prävention und zur Behandlung kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, stellt eine günstige Kombination mit verstärkter Wirkung und vermindertem Wirkstoffeinsatz dar.

So führen NHE-Inhibitoren zu einen wirksamen Schutz gegen Endothelschädigungen unterschiedlicher Genese. Mit diesem Schutz der Gefäße gegen das Syndrom der endothelialen Dysfunktion sind NHE-Inhibitoren wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem eignen sich NHE-Inhibitoren zur Behandlung des nicht-insulinabhängigen Diabetes (NIDDM), wobei beispielweise die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR-Agonisten, wie Rosiglitazone, Pioglitazone etc, mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken NHE-Inhibitoren der Entstehung diabetischer Spätkomplikation entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den oben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

NHE-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die auch unabhängig von akuten Mangeldurchblutungszuständen sind und auch bei normalen, nicht-ischämischen Bedingungen auftreten können. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. NHE-Inhibitoren eignen sich somit hervorragend zur Behandlung und Prävention der altersbedingt fortschreitenden endothelialen Dysfunktion, insbesondere von claudicatio intermittens. Außerdem eignen sich NHE-Inhibitoren somit hervorragend zur Behandlung und Prävention der Herzinsuffizienz, des congestive heart failure (CHF) sowie zur Behandlung und insbesondere zur Prävention von altersbedingten Formen von Krebs.
Dabei kommt eine Kombination mit blutdrucksenkenden Medikamenten, wie mit ACE-Hemmern, Angiotensinrezeptorantagonisten, Diuretika, Ca²⁺-Antagonisten etc. oder mit stoffwechselnormalisierenden Medikamenten, wie Cholesterinsenkern, in Betracht. Die Verbindungen der Formel I eignen sich somit zur Prävention altersbedingter Gewebsveränderungen und zur Gesunderhaltung und Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Weiterhin sind NHE-Inhibitoren zur Behandlung von durch Bakterien sowie durch Protozoen ausgelösten Erkrankungen (human und veterinär) geeignet. Bei den durch Protozoen ausgelösten Erkrankungen sind insbesondere Malariaerkrankungen des Menschen und Hühnercoccidiose zu nennen. Außerdem eignen sich die Verbindungen als Mittel zur Bekämpfung von saugenden Parasiten in der Human- und Veterinärmedizin sowie im Pflanzenschutz. Dabei ist die Verwendung als Mittel gegen blutsaugende Parasiten in der Human- und Veterinärmedizin bevorzugt.

Die genannten Verbindungen finden daher vorteilhaft Verwendung allein oder in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

Die Erfindung betrifft weiterhin die Verwendung der Verbindungen der Formel I und deren pharmazeutisch verträgliche Salze zur Verwendung als Medikament.

Die Erfindung betrifft auch Heilmittel/pharmazeutische Zubereitungen für die humane, veterinäre oder phytoprotektive Anwendung enthaltend eine wirksame Menge einer Verbindung der Formel I und / oder eines pharmazeutisch verträglichen Salzes davon, ebenso wie pharmazeutische Zubereitungen für die humane, veterinäre oder phytoprotektive Anwendung enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.

Arzneimittel, die eine Verbindung der Formel I oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin und im Pflanzenschutz. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.
Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v.-Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

### Versuchsbeschreibungen und Beispiele

Liste der verwendeten Abkürzungen:
- Fp.: Schmelzpunkt
- MPRC: Cartridge L-026-30; SI60 40-63µm; Super Vario Flash; max.press. 3 bar Götec-Labortechnik GmbH
- MPLC: Mitteldruck-Flüssigkeitschromatographie
- THF: Tetrahydrofuran
- RT: Raumtemperatur

### Beispiel 1: 1-Amino-4-(4-aminophenyl)-6-chlor-3,4-dihydroisochinolin Hydrochlorid (1A)und 1-Amino-4-(4-aminophenyl)-3,4-dihydroisochinolin Hydrochlorid (1B)

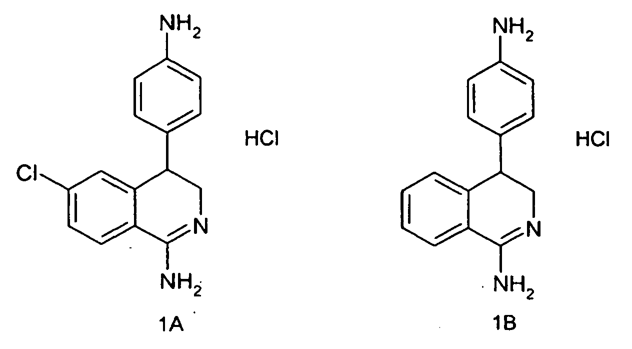

### a) 3'-Chlor-4-nitrodiphenylacetonitril

Eine Mischung aus 3 g 3-Chlorbenzylcyanid, 3,1 g 1-Chlor-4-nitrobenzol und 4,3 g N-Benzyl-N,N,N-triethylammonium-chlorid in 5ml THF wurde ca. 10 min bei Raumtemperatur gerührt und sodann eine Lösung aus 10,1 ml 50%ige wässrige NaOH-Lösung zugetropft. Man rührte die blaugrüne Lösung 3 Stunden bei 60°C, verdünnte mit etwas Wasser und stellte nach dem Abkühlen auf Raumtemperatur mit 36%iger konzentrierter Salzsäure sauer. Man extrahierte mit Ethylacetat, wusch die organische Phase mit Wasser und destillierte das Lösungsmittel ab. Mit nachfolgender Mitteldruck-Flüssigkeitschromatografie (MPLC) an der MPRC-Säule mit einer Mischung aus Ethylacetat und n-Heptan (2:1) isolierte man das ölig-amorphe Produkt.

### b) 2-(3-Chlorphenyl)-2-(4-nitrophenyl)-ethylamin

Zu 15,8 mmol (15,8 ml) Boran-Tetrahydrofuran-Komplex tropfte man eine Lösung aus 13,17 mmol 3'-Chlor-4-nitrodiphenylacetonitril in wasserfreiem THF. Nach 4-stündigem Rühren bei Raumtemperatur und weiterem Stehenlassen für 15 Stunden bei RT destillierte man das Lösungsmittel am Rotavapor unter vermindertem Druck ab, versetzte den Rückstand mit Ethanol und einigen Tropfen 36%iger, konzentrierte Salzsäure, erhitzte für ca. 30 Minuten auf dem Dampfbad und destillierte sodann das ethanolische Solvens ab. Der grüne ölige Rückstand wurde mit Wasser versetzt, mit 2n NaOH alkalisch gestellt, mit Ethylacetat extrahierte, und nach dem Waschen der organischen Phase getrocknet und das Lösungsmittel am Rotavapor abdestilliert. Durch MPLC-Säulenchromatographie mit einer MPRC und einem Gemisch aus Dichlormethan und Methanol (10:1) erhielt man das gewünschte Produkt als grünliches amorphes Öl.

### c) 2-(3-Chlorphenyl)-2-(4-nitrophenyl)-ethylisothiocyanat

Zu einer wässrigen Lösung aus 2,71 mmol Natriumbicarbonat gab man eine Lösung aus 1,192 mmol Thiophosgen in 5 ml Merthylenchlorid. Zu dieser Mischung tropfte man nun innerhalb von 15 Minuten eine Mischung aus 1,084 mmol 2-(3-Chlorphenyl)-2-(4-nitrophenyl)-ethylamin. Nach dem Abtrennen der organischen Phase und nochmaliger Extraktion der wässrigen Phase mit Dichlormethan wusch man die vereinigten organischen Phasen mit Wasser und destillierte das Lösungsmittel am Rotavapor unter vermindertem Druck ab. Das rötliche, ölige 2-(3-Chlorphenyl)-2-(4-nitrophenyl)-ethylisothiocyanat wurde im Kühlschrank bis zur weiteren Verarbeitung unter Ausschluss von Feuchtigkeit verschlossen aufbewahrt.

### d) 6-Chlor-1-mercapto-4-(4-nitrophenyl)-3,4-dihydroisochinolin

Unter externer Eiskühlung und interner Rührung gab man 1,75 ml konzentrierte Schwefelsäure zu 0,4g (1,255 mmol) 2-(3-Chlorphenyl)-2-(4-nitrophenyl)-ethylisothiocyanat. Nach weiteren 4 Stunden Rühren bei RT versetzte man mit Eiswasser und extrahierte mit Dichlormethan.

### e) 6-Chlor-1-methylthio-4-(4-nitrophenyl)-3,4-dihydroisochinolin

Durch Reaktion von 0,48 g 6-Chlor-1-mercapto-4-(4-nitrophenyl)-3,4-dihydroisochinolin in 20 ml Aceton mit 0,85g Methyljodid über 3 Stunden und anschließendem Abdestillieren des Acetons erhielt man 6-Chlor-1-methylthio-4-(4-nitrophenyl)-3,4-dihydroisochinolin-hydrojodid als gelben Feststoff.
Fp.: 177-182°C.
Die korrespondierende freie Base 6-Chlor-1-methylthio-4-(4-nitrophenyl)-3,4-dihydroisochinolin konnte aus dem Hydrojodid mit wässriger 2n NaOH freigesetzt und mit Ethylacetat extrahiert werden. Teilweise festes Produkt.

### f) 1-Amino-6-chlor-4-(4-nitrophenyl)-3,4-dihydroisochinolin

Eine Lösung aus 0,45g 6-Chlor-1-methylthio-4-(4-nitrophenyl)-3,4-dihydroisochinolin und 30 ml Ammoniak-gesättigter THF-Lösung wurde im Autoklaven über 10 Stunden auf 80°C und anschließend über weitere 15 Stunden auf 120°C erhitzt. Die anschließende MPLC auf einer MPRC-Kartusche und einem Gemisch aus 20 Volumenteilen Ethylacetat : 10 Volumenteilen n-Heptan : 3 Volumenteilen Eisessig führt zur Elution von Ausgangsmaterial (6-Chlor-1-methylthio-4-(4-nitrophenyl)-3,4-dihydroisochinolin). Durch anschließende Elution mit Methanol erhielt man das 1-Amino-6-chlor-4-(4-nitrophenyl)-3,4-dihydroisochinolin.
Fp.: 170-185°C.

### g) 1-Amino-4-(4-aminophenyl)-6-chlor-3,4-dihydroisochinolin Hydrochlorid (1A) und 1-Amino-4-(4-aminophenyl)- 3,4-dihydroisochinolin Hydrochlorid (1B)

Eine Mischung von 210 mg 1-Amino-6-chlor-4-(4-nitrophenyl)-3,4-dihydroisochinolin, 10 ml Ethanol, 1 ml konzentrierte Salzsäure (36%) und 87mg Platin-IV-oxid wurden bei Raumtemperatur und Normaldruck bis zum Ende der Wasserstoffaufnahme hydriert. Nach dem Abdestillieren des Lösungsmittels trennte man 2 Komponenten durch MPLC an einer MPRC-Kartusche und mit einer Mischung aus 10 Volumenteilen Ethylacetat, 5 Volumenteilen Methanol, 5 Volumenteilen n-Heptan, 5 Volumenteilen Dichlormethan und 1 Volumenteil Ammoniak (konzentriert).
Das Produkt wurde in wenig Essigester gelöst, mit etherischer HCl sauer gestellt, bei Raumtemperatur gerührt und abgesaugt. Man erhielt 1-Amino-4-(4-aminophenyl)-6-chlor-3,4-dihydroisochinolin Hydrochloride (1A) als farblose kristalline Substanz.
Rf = 0.16 (silica gel, Eisessigester/ Methanol/Heptan/Dichloromethan/Ammoniak 10:5:5:5:1)
Fp.: 278-284°C
MS m/z = 271 (m)+
Das Produkt wurde auch in wenig Essigester gelöst, mit etherischer HCl sauer gestellt, bei Raumtemperatur gerührt und abgesaugt. Man erhielt 1-Amino-4-(4-aminophenyl)-3,4-dihydroisochinolin Hydrochloride (1B) als farblose Kristalline Substanz.
Rf = 0.11 (silica gel, Eisessigester/ Methanol/Heptan/Dichloromethan/Ammoniak 10:5:5:5:1)
MS m/z = 237 (m)+

### Beispiel 2: 1-Amino-4-(2-aminophenyl)-6-chlor-3,4-dihydroisochinolin Hydrochlorid

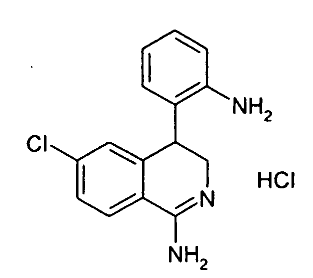

### a) 3'-Chlor-2-nitrodiphenylacetonitril

erhielt man analog der in Beispiel 1 a) angegebenen Vorschrift aus 3-Chlorbenzylcyanid, 2-Fluornitrobenzol in Gegenwart von N-Benzyl-N,N,N-triethylammoniumchlorid als Zweiphasenkatalysator als ölig-amorphes Produkt.

### b) 2-(3-Chlorphenyl)-2-(2-nitrophenyl)-ethylamin Acetat

erhielt man analog der in Beispiel 1 b) angegebenen Vorschrift durch selektive Reduktion der Nitril Gruppe mit BH₃-THF-Komplex.

### c) 2-(3-Chlorphenyl)-2-(2-nitrophenyl)-ethylisothiocyanat

erhielt man analog der in Beispiel 1 c) angegebenen Vorschrift aus 2-(3-Chlorphenyl)-2-(2-nitrophenyl)-ethylamin Acetat und Thiophosgen in Dichlormethan und analoger Aufbewahrung bis zur weiteren Umsetzung unter Inertgas im Kühlschrank.

### d) 6-Chlor-1-mercapto-4-(2-nitrophenyl)-3,4-dihydroisochinolin

erhielt man analog der in Beispiel 1 d) angegebenen Vorschrift aus 2-(3-Chlorphenyl)-2-(2-nitrophenyl)-ethylisothiocyanat und konzentrierter Schwefelsäure als amorphes, halbfestes Produkt.

### e) 6-Chlor-1-methylthio-4-(2-nitrophenyl)-3,4-dihydroisochinolin

erhielt man analog der in Beispiel 1 e) angegebenen Vorschrift aus 6-Chlor-1-mercapto-4-(2-nitrophenyl)-3,4-dihydroisochinolin und Methyljodid und anschließender Behandlung mit 2n Natronlauge. Gelbes amorphes Öl.

### f) 1-Amino-6-chlor-4-(2-nitrophenyl)-3,4-dihydroisochinolin Hydrochlorid

erhielt man analog der in Beispiel 1 f) angegebenen Vorschrift aus 6-Chlor-1-methylthio-4-(2-nitrophenyl)-3,4-dihydroisochinolin als amorphes Produkt, das ohne weitere Reinigungsoperationen in Stufe g) weiter umgesetzt wurde.

### g) 1-Amino-4-(2-aminophenyl)-6-chlor-3,4-dihydroisochinolin Hydrochloride

erhielt man analog der in Beispiel 1 g) angegebenen Vorschrift aus 1-Amino-6-chlor-4-(2-nitrophenyl)-3,4-dihydroisochinolin Hydrochlorid durch Hydrierung mit PtO₂ und MPLC und einem Elutionsgemisch aus 10 Volumenteilen Ethylacetat, 5 Volumenteilen n-Heptan, 5 Volumenteilen Dichlormethan, 5 Volumenteilen Methanol und 1 Volumenteil konzentrierter wässriger Ammoniaklösung. Das Produkt wurde in wenig Essigester gelöst, mit etherischer HCl sauer gestellt, bei Raumtemperatur gerührt und abgesaugt.
Zersetzungspunkt ab 175 °C (Aufschäumen).

### Beispiel 3: 4-(4-Aminophenyl)-6-chlor-1-methylamino-3,4-dihydroisochinolin Hydrochlorid

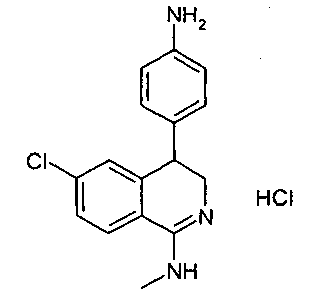

### a) 6-Chlor-1-methylamino-4-(4-nitrophenyl)-3,4-dihydroisochinolin

400mg 6-Chlor-1-methylthio-4-(4-nitrophenyl)-3,4-dihydroisochinolin (siehe Verbindung 1 e) wurden mit 2g Methylamin in THF im Schüttelautoklaven unter Inertgas über 20 Stunden auf 120°C erhitzt. Nach Abdestillieren des Lösungsmittels wurde eine MPLC auf einer MPRC-Kartusche mit einem Gemisch aus 10 Volumenteilen Ethylacetat, 5 Volumenteilen n-Heptan, 5 Volumenteilen Dichlormethan, 5 Volumenteilen Methanol und 1 Volumenteil konzentrierter wässriger Ammoniaklösung chromatographiert. Feststoff, Fp.; 60-65°C (amorphes Produkt)

### b) 6-Chlor-1-methylamino-4-(4-nitrophenyl)-3,4-dihydroisochinolin-hydrochlorid

erhielt man durch Stehenlassen einer Ethylacetat-Lösung von 15mg 6-Chlor-1-methylamino-4-(4-nitrophenyl)-3,4-dihydroisochinolin nach Zugabe einer gesättigten Lösung von Chlorwasserstoffgas in Diethylether. 6-Chlor-1-methylamino-4-(4-nitrophenyl)-3,4-dihydroisochinolin-hydrochlorid kristallisierte nach kurzem Erwärmen aus der Lösung als kristallines farbloses Produkt aus und wurde abfiltriert. Fp.: 282-285°C

### c) 4-(4-Aminophenyl)-6-chlor-1-methylamino-3,4-dihydroisochinolin

Zu einer Lösung von 255mg 6-Chlor-1-methylamino-4-(4-nitrophenyl)-3,4-dihydroisochinolin in 4,5-5 ml Eisessig gab man 135,2 mg Eisenpulver, tropfte anschließend 1,8 ml konzentrierte Salzsäure zu und kochte 2 Stunden unter Rückflussbedingungen. Man destillierte das Lösungsmittel ab, versetzte den Rückstand mit Wasser und stellte mit 2n NaOH alkalisch. Diese wässrige Phase wurde mit Ethylacetat extrahiert und durch MPLC unter Verwendung einer MPRC-Kartusche mit einem Gemisch aus 10 Volumenteilen Ethylacetat, 5 Volumenteilen n-Heptan, 5 Volumenteilen Dichlormethan, 5 Volumenteilen Methanol und 1 Volumenteil konzentrierter wässriger Ammoniaklösung chromatographisch gereinigt. Amorphes Produkt.

### d) 4-(4-Aminophenyl)-6-chlor-1-methylamino-3,4-dihydroisochinolin Hydrochlorid

erhielt man analog der in Beispiel 3b angegebenen Vorschrift aus einer Ethylacetat-Lösung von 4-(4-Aminophenyl)-6-chlor-1-methylamino-3,4-dihydroisochinolin mit etherischer Chlorwasserstofflösung. Farblose kristalline Substanz. Fp.: 306-310°C.

### Beispiel 4: 4-(4-Aminophenyl)-6-chlor-1-dimethylamino-3,4-dihydroisochinolin Hydrochlorid

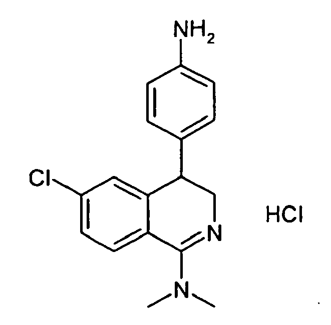

### a) 6-Chlor-1-dimethylamino-4-(4-nitrophenyl)-3,4-dihydroisochinolin

400mg 6-Chlor-1-methylthio-4-(4-nitrophenyl)-3,4-dihydroisochinolin (Verbindung 1) wurden mit 20 ml Dimethylamin in THF im Schüttelautoklaven unter Inertgas über 20 Stunden auf 120°C erhitzt. Nach Abdestillieren des Lösungsmittels wurde eine MPLC auf einer MPRC-Kartusche mit einem Gemisch aus 10 Volumenteilen Ethylacetat, 5 Volumenteilen n-Heptan, 5 Volumenteilen Dichlormethan, 5 Volumenteilen Methanol und 1 Volumenteil konzentrierter wässriger Ammoniaklösung chromatographiert. Halbfestes, teils amorphes Produkt.

### b) 4-(4-Aminophenyl)-6-chlor-1-dimethylamino-3,4-dihydroisochinolin Hydrochlorid

Zu einer Lösung von 195mg 6-Chlor-1-dimethylamino-4-(4-nitrophenyl)-3,4-dihydroisochinolin in 3,5ml Eisessig gab man 100 mg Eisenpulver, tropfte anschließend 1,8 ml konzentrierte Salzsäure zu und kochte 2 Stunden unter Rückflussbedingungen. Man destillierte das Lösungsmittel ab, versetzte den Rückstand mit Wasser und stellte mit 2n NaOH alkalisch. Diese wässrige Phase wurde mit Ethylacetat extrahiert und durch MPLC unter Verwendung einer MPRC-Kartusche mit einem Gemisch aus 10 Volumenteilen Ethylacetat, 5 Volumenteilen n-Heptan, 5 Volumenteilen Dichlormethan, 5 Volumenteilen Methanol und 1 Volumenteil konzentrierter wässriger Ammoniaklösung chromatographisch gereinigt. Nach Abdestillieren des Lösungsmittels am Rotavapor wurde der zähe amorphe Rückstand in Essigester mit etherischer Chlorwasserstofflösung behandelt und der kristalline Feststoff abfiltriert.
Fp.: 255-260°C.

### Beispiel 5: 4-(4-Aminophenyl)-6-chlor-1-(N-pyrrolidino)-3,4-dihydroisochinolin Hydrochlorid

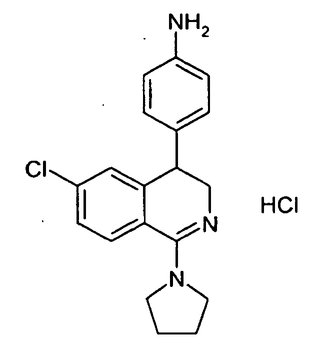

### a) 6-Chlor-4-(4-nitrophenyl)-1-(N-pyrrolidino)-3,4-dihydroisochinolin

300mg 6-Chlor-1-methylthio-4-(4-nitrophenyl)-3,4-dihydroisochinolin (Verbindung 1) wurden mit 0,151 ml Pyrrolidin über 3 Stunden auf 120°C erhitzt. Nach Abdestillieren des Lösungsmittels wurde eine MPLC auf einer MPRC-Kartusche mit einem Gemisch aus gleichen Volumenteilen Dichlormethan und Methanol chromatographiert. Halbfestes, teils kristallines Produkt.

### b) 4-(4-Aminophenyl)-6-chlor-1-(N-pyrrolidino)-3,4-dihydroisochinolin

Zu einer Lösung von 171,2mg 6-Chlor-1-(N-pyrrolidino)-4-(4-nitrophenyl)-3,4-dihydroisochinolin in 2,8ml Eisessig gab man 80,6 mg Eisenpulver, tropfte anschließend 1,8 ml konzentrierte Salzsäure zu und kochte 2 Stunden unter Rückflussbedingungen. Man destillierte das Lösungsmittel ab, versetzte den Rückstand mit Wasser und stellte mit 2n NaOH alkalisch. Diese wässrige Phase wurde mit Ethylacetat extrahiert und durch MPLC unter Verwendung einer MPRC-Kartusche mit einem Gemisch aus 10 Volumenteilen Ethylacetat, 5 Volumenteilen n-Heptan, 5 Volumenteilen Dichlormethan, 5 Volumenteilen Methanol und 1 Volumenteil konzentrierter wässriger Ammoniaklösung chromatographisch gereinigt. Teilweise amorphes Produkt.

### c) 4-(4-Aminophenyl)-6-chlor-1-(N-pyrrolidino)-3,4-dihydroisochinolin Hydrochlorid

erhielt man analog der in Beispiel 3b angegebenen Vorschrift aus einer Ethylacetat-Lösung von 4-(4-Aminophenyl)-6-chlor-1-(N-pyrrolidino)-3,4-dihydroisochinolin mit etherischer Chlorwasserstofflösung. Farblose kristalline Substanz. Schmelzen unter Zersetzung ab 250°C.

### Beispiel 6: N-(Ethoxycarbonymethyl)-N'-4-[(6-chloro-3,4-dihydro-1-dimethylaminoisochinolin-4-yl)phenyl]harnstoff Hydrochlorid

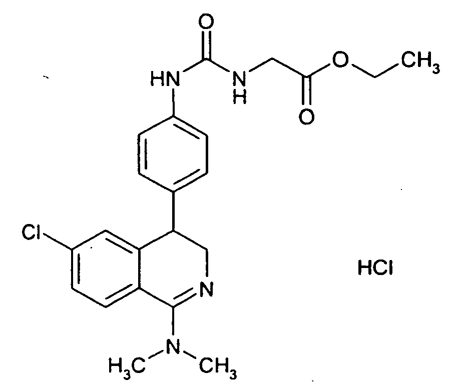

### a) 6-Chloro-1-dimethylamino-4-(4-nitrophenyl)-3,4-dihydrochinolin

400 mg 6-Chlor-1-methylthio-4-(4-nitrophenyl)-3,4-dihydroisochinolin (Verbindung 1) wurden mit einer 2M Lösung THF/Dimethylamine (20 ml) im Schüttelautoklaven über 20 Stunden auf 120°C erhitzt. Nach Abdestillieren des Lösungsmittels wurde eine MPLC auf einer MPRC-Kartusche mit einem Gemisch aus gleichen Volumenteilen Dichlormethan und Methanol chromatographiert. Man erhielt einen semikristallinen Feststoff.

### b) 4-(4-aminophenyl)-6-chlor-1 (dimethylamino)-3,4-dihydroisochinolin

Zu einer Lösung von 195 mg 6-Chloro-1-dimethylamino-4-(4-nitrophenyl)-3,4-dihydrochinolin in 3ml Eisessig gab man 99 mg Eisenpulver, tropfte anschließend 1,8 ml konzentrierte Salzsäure zu und kochte 2 Stunden unter Rückflussbedingungen. Man destillierte das Lösungsmittel ab, versetzte den Rückstand mit Wasser und stellte mit 2n NaOH alkalisch. Diese wässrige Phase wurde mit Ethylacetat extrahiert und durch MPLC unter Verwendung einer MPRC-Kartusche mit einem Gemisch aus 10 Volumenteilen Ethylacetat, 5 Volumenteilen n-Heptan, 5 Volumenteilen Dichlormethan, 5 Volumenteilen Methanol und 1 Volumenteil konzentrierter wässriger Ammoniaklösung chromatographisch gereinigt. Der erhaltene zähe Rückstand wird in Essigester suspendiert und mit Ether/HCl sauer gestellt. Der kristalline Feststoff wurde abfiltriert.
Schmelzen unter Zersetzung ab 250°C.

### c) N-(ethoxycarbonymethyl)-N'-4-[(6-chlor-3,4-dihydro-1-dimethylaminoisochinolin-4-yl)phenyl]harnstoff

Durch Reaktion von 76 mg 4-(4-Aminophenyl)-6-chlor-1 (dimethylamino)-3,4-dihydroisochinolin in 6 ml Dichlormethan mit 33 mg Ethylisocyanatoacetat in 3 ml Dichlormethan über 3 Stunden und anschließendem Abdestillieren des Dichlormethans erhielt man N-(Ethoxycarbonymethyl)-N'-4-[(6-chlor-3,4-dihydro-1-dimethylaminoisochinolin-4-yl)phenyl]harnstoff als dunkelgelben, festen Rückstand. Die anschließende MPLC auf einer MPRC-Kartusche mit einem Gemisch aus 10 Volumenteil Essigester, 5 Volumenteil n-Heptan, 5 Volumenteil Dichlormethan, 5 Volumenteil Methanol und 1 Volumenteil konzentrierter wässriger Ammoniaklösung führte zur Eluierung von Produkt. Das Produkt wurde mit wenig Essigester versetzt und mit etherische HCl sauer gestellt.
Fp.: 120°C.

### Beispiel 7: 3-{4-[(6-Chlor-3,4-dihydro-1-dimethylaminoisochinolin-4-yl)phenyl]}-imidazolidin-2,4-dion Hydrochlorid

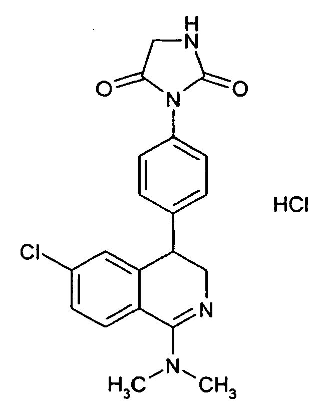

43 mg N-(Ethoxycarbonymethyl)-N'-4-[(6-chlor-3,4-dihydro-1-dimethylamino-isochinolin-4-yl)phenyl]harnstoff (Beispiel 6) wurden in 5 ml halbkonzentrierter HCl 6 Stunden unter Rückfluss gekocht. Nach 1 Stunde Rühren bei Raumtemperatur destillierte man das Wasser ab. Der Rückstand wurde mit wenig Wasser versetzt und mit gesättigter K₂CO₃ Lösung neutral gestellt. Es fällt ein Feststoff aus und wurde durch MPLC unter Verwendung einer MPRC-Kartusche mit einem Gemisch aus 10 Volumenteil Essigester, 5 Volumenteil n-Heptan, 5 Volumenteil Dichlomethan, 6 Volumenteil Methanol und 1 Volumenteil konzentrierter wässriger Ammoniaklösung chromatographisch gereinigt. Das Produkt wurde in wenig Essigester gelöst, mit etherischer HCl sauer gestellt, bei Raumtemperatur gerührt und abgesaugt.
Fp.: ab 140°C, beginnende Sublimation >310°C.

### Pharmakologische Daten:

### Testbeschreibung: Bestimmung der NHE-inhibitorischen Wirkung

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wurde die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl -Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wurde ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wurde ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM Carl₂, 1,25 mM MgCl₂, 0,97 mM K₂HPO₄, 0,23 mM KH₂PO₄, 5 mM Hepes, 5 mM Glucose, mit 1 M NaOH wurde ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

Die inhibitorische Wirkung (IC₅₀-Werte) diverser Beispielverbindungen auf den NHE3 ist in nachfolgender Tabelle aufgeführt:

| Beispiel | IC₅₀ [µM] |
|---|---|
| 1A | 1,95 |
| 1 B | 8,24 |
| 2 | 1,63 |
| 3 | 4,93 |
| 4 | 10 |
| 5 | 10 |
| 6 | 10 |

## Patentansprüche

1. Verbindungen der Formel I 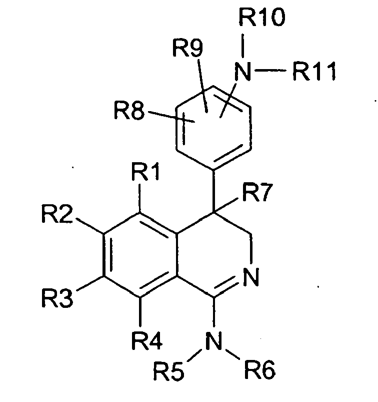 worin bedeuten:
R1, R2, R3 und R4
unabhängig voneinander Wasserstoff, F, Cl, Br, I, CN, NO₂, CF₃, CH₃-SO₂, Alkyl mit 1, 2, 3 oder 4 C-Atomen, NH₂, NH-CH₃ oder N(CH₃)₂;
R5 und R6
unabhängig voneinander Wasserstoff, Alkyl mit 1, 2, 3, 4, 5 oder 6 C-Atomen, CF₃-CH₂-, Cycloalkyl mit 3, 4, 5 oder 6 C-Atomen oder Cyclopropyl-CH₂-,
oder
R5 und R6
bilden gemeinsam mit dem Stickstoffatom, an das sie gebunden sind, einen 4-, 5-, 6-, 7-, 8-, 9- oder 10-gliedrigen Ring, in dem ein oder zwei CH₂-Gruppen unabhängig voneinander durch NR12, Schwefel, Sauerstoff, C(O) oder SO₂ ersetzt sein können;
R12 Wasserstoff, Alkyl mit 1, 2, 3 oder 4 C-Atomen oder Cycloalkyl mit 3, 4, 5 oder 6 C-Atomen;
R7 Wasserstoff oder Alkyl mit 1, 2, 3 oder 4 C-Atomen;
R8 und R9
unabhängig voneinander Wasserstoff, F, Cl, Br, OH, Alkyl mit 1, 2, 3 oder 4 C-Atomen, CH₃O, CF₃, oder CH₃SO₂;
R10 und 11
unabhängig voneinander R13-(CₘH₂ₘ)-Bₙ, wobei
m Null, 1, 2, 3 oder 4;
n Null oder 1;
B -CO-, -CONR14- oder -SO₂-;
R14 Wasserstoff oder Alkyl mit 1, 2, 3, 4, 5 oder 6 C-Atomen;
R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl), 1-Morpholinyl, -COOR15, OR16, NR17R18 oder Phenyl, das unabhängig voneinander 1 oder 2 Substituenten trägt, ausgewählt aus der Gruppe Chlor, Fluor, Methyl und Methoxy;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei, drei oder vier CH₂-Gruppen unabhängig voneinander durch NR19, Schwefel, Sauerstoff, C(O) oder SO₂ ersetzt sein können;
R19 Wasserstoff, Alkyl mit 1, 2, 3 oder 4 C-Atomen oder Cycloalkyl mit 3, 4, 5 oder 6 C-Atomen;
sowie deren pharmazeutisch verträgliche Salze und Trifluoracetate.

2. Verbindungen der Formel I nach Anspruch 1, worin bedeuten
R1, R2, R3 und R4
unabhängig voneinander Wasserstoff, F, Cl, Br, CN, CF₃, CH₃-SO₂, Alkyl mit 1, 2, 3 oder 4 C-Atomen, NH₂, NH-CH₃ oder N(CH₃)₂;
R5 und R6
unabhängig voneinander Wasserstoff, Alkyl mit 1, 2, 3, 4, 5 oder 6 C-Atomen, CF₃-CH₂-, Cycloalkyl mit 3, 4, 5 oder 6 C-Atomen oder Cyclopropyl-CH₂-,
oder
R5 und R6 bilden gemeinsam mit dem Stickstoffatom, an das sie gebunden sind, einen 4-, 5-, 6-, 7-, 8-, 9- oder 10-gliedrigen Ring;
R7 Wasserstoff oder Methyl;
R8 und R9
unabhängig voneinander Wasserstoff, F, Cl, OH, Alkyl mit 1, 2, 3 oder 4 C-Atomen, CH₃O CF₃, oder CH₃SO₂;
R10 und 11
unabhängig voneinander R13-(CₘH₂ₘ)-Bₙ, wobei
m Null, 1, 2, 3 oder 4;
n Null oder 1;
B -CO-, -CONR14- oder -SO₂-;
R14 Wasserstoff oder Alkyl mit 1, 2, 3, 4, 5 oder 6 C-Atomen;
R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl),1-Morpholinyl, -COOR15, OR16, NR17R18 oder Phenyl, das unabhängig voneinander 1 oder 2 Substituenten trägt, ausgewählt aus der Gruppe Chlor, Fluor, Methyl und Methoxy;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei, drei oder vier CH₂-Gruppen unabhängig voneinander durch NR19, Schwefel, Sauerstoff, C(O) oder SO₂ ersetzt sein können;
R19 Wasserstoff oder Alkyl mit 1, 2, 3 oder 4 C-Atomen;
sowie deren pharmazeutisch verträgliche Salze und Trifluoracetate.

3. Verbindungen der Formel I nach Anspruch 1 und/oder 2, worin bedeuten
R1, R2, R3 und R4
unabhängig voneinander Wasserstoff, F, CI, Br, CN, CF₃, CH₃-SO₂, Methyl, Ethyl, NH₂, NH-CH₃ oder N(CH₃)₂;
R5 und R6
unabhängig voneinander Wasserstoff, Methyl, Ethyl, Isopropyl, CF₃-CH₂ oder Cycloalkyl mit 3, 4, 5 oder 6 C-Atomen;
oder
R5 und R6
bilden gemeinsam mit dem Stickstoffatom, an das sie gebunden sind, einen 4-, 5-, 6-, 7-, 8-, 9- oder 10-gliedrigen Ring;
R7 Wasserstoff oder Methyl;
R8 und R9
unabhängig voneinander Wasserstoff, Cl oder Methyl;
R10 und 11
unabhängig voneinander R13-(CₘH₂ₘ)-Bₙ, wobei
m Null, 1, 2, 3 oder 4;
n Null oder 1;
B -CO-, -CONR14- oder -SO₂-;
R14 Wasserstoff oder Alkyl mit 1, 2, 3, 4, 5 oder 6 C-Atomen;
R13 Wasserstoff, Methyl, Ethyl, Isopropyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen unabhängig voneinander durch NR19, oder C(O) ersetzt sein können; R19 Wasserstoff oder Methyl;
sowie deren pharmazeutisch verträgliche Salze und Trifluoracetate.

4. Verbindungen der Formel I nach einem oder mehreren der Ansprüche 1 bis 3, worin bedeuten
R1 und R3
Wasserstoff;
R2 und R4
unabhängig voneinander Wasserstoff oder Cl;
R5 und R6
unabhängig voneinander Wasserstoff oder Methyl;
oder
R5 und R6
bilden gemeinsam mit dem Stickstoffatom, an das sie gebunden sind, einen 5-oder 6-gliedrigen Ring;
R7 Wasserstoff;
R8 und R9
unabhängig voneinander Wasserstoff oder Cl;
R10 und 11
unabhängig voneinander R13-(CₘH₂ₘ)-Bₙ, wobei
m Null, 1, 2, 3 oder 4;
n Null oder 1;
B -CO- oder -CONR14-;
R14 Wasserstoff oder Methyl;
R13 Wasserstoff, Methyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen unabhängig voneinander durch NR19, oder C(O) ersetzt sein können; R19 Wasserstoff oder Methyl;
sowie deren pharmazeutisch verträgliche Salze und Trifluoracetate.

5. Verbindungen der Formel I nach einem oder mehreren der Ansprüche 1 bis 4 ausgewählt aus der Gruppe
1-Amino-4-(4-aminophenyl)-6-chlor-3,4-dihydroisochinolin,
1-Amino-4-(4-aminophenyl)- 3,4-dihydroisochinolin,
1-Amino-4-(2-aminophenyl)-6-chlor-3,4-dihydroisochinolin,
4-(4-Aminophenyl)-6-chlor-1-methylamino-3,4-dihydroisochinolin,
4-(4-Aminophenyl)-6-chlor-1-dimethylamino-3,4-dihydroisochinolin,
4-(4-Aminophenyl)-6-chlor-1-(N-pyrrolidino)-3,4-dihydroisochinolin,
N-(Ethoxycarbonymethyl)-N'-4-[(6-chloro-3,4-dihydro-1-dimethylaminoisochinolin-4-yl)phenyl]harnstoff
und
3-{4-[(6-Chlor-3,4-dihydro-1-dimethylaminoisochinolin-4-yl)phenyl]}- imidazolidin-2,4-dion
sowie deren pharmazeutisch verträgliche Salze und Trifluoracetate.

6. Verbindungen der Formel I und deren pharmazeutisch verträgliche Salze nach einem oder mehreren der Ansprüche 1 bis 5 zur Verwendung als Medikament.

7. Verwendung einer Verbindung der Formel I und/oder dessen pharmazeutisch verträglicher Salze nach einem oder mehreren der Ansprüche 1 bis 5 zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, degenerative Erkrankungen des ZNS, Verminderung der Gedächtnisleistung, Demens und Alzheimersche Erkrankung, und von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, von Schockzuständen oder der Zuckerkrankheit und diabetischer Spätschäden oder von Krankheiten, bei denen die Zellproliferation eine primäre oder sekundäre Ursache darstellt, zur Konservierung und Lagerung von Transplantaten für chirurgische Maßnahmen, für den Einsatz bei chirurgischen Operationen und Organtransplantationen und zur Gesunderhaltung und Lebensverlängerung.

8. Verwendung einer Verbindung der Formel I und/oder dessen pharmazeutisch verträglicher Salze nach einem oder mehreren der Ansprüche der Ansprüche 1 bis 5 in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, von Schockzuständen oder der Zuckerkrankheit und diabetischer Spätschäden oder von Krankheiten, bei denen die Zellproliferation eine primäre oder sekundäre Ursache darstellt zur Konservierung und Lagerung von Transplantaten für chirurgische Maßnahmen, für den Einsatz bei chirurgischen Operationen und Organtransplantationen und zur Gesunderhaltung und Lebensverlängerung.

9. Verwendung einer Verbindung der Formel I und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe von Störungen des Atemantriebs und/oder von Schlaf-bedingten Atemstörungen wie Schlafapnoen.

10. Verwendung einer Verbindung der Formel I und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe des Schnarchens.

11. Verwendung einer Verbindung der Formel I und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder zur Prophylaxe von akuten oder chronischen Nierenerkrankungen, des akuten Nierenversagens oder des chronischen Nierenversagens.

12. Verwendung einer Verbindung der Formel I und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe von Störungen der Darmfunktion.

13. Pharmazeutische Zubereitung für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 5.

14. Pharmazeutische Zubereitung für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 5, in Kombination mit anderen pharmakologischen Wirkstoffen oder Arzneimitteln.

## Claims

1. A compound of the formula I 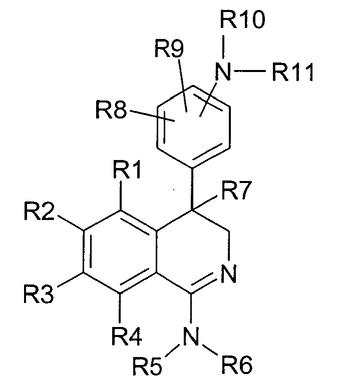 in which the meanings are:
R1, R2, R3 and R4
independently of one another hydrogen, F, Cl, Br, I, CN, NO₂, CF₃, CH₃-SO₂, alkyl having 1, 2, 3 or 4 C atoms, NH₂, NH-CH₃ or N(CH₃)₂;
R5 and R6
independently of one another hydrogen, alkyl having 1, 2, 3, 4, 5 or 6 C atoms, CF₃-CH₂-, cycloalkyl having 3, 4, 5 or 6 C atoms or cyclopropyl-CH₂-,
or
R5 and R6
form together with the nitrogen atom to which they are bonded a 4-, 5-, 6-, 7-, 8-, 9- or 10-membered ring in which one or two CH₂ groups may be replaced independently of one another by NR12, sulfur, oxygen, C(O) or SO₂;
R12hydrogen, alkyl having 1, 2, 3 or 4 C atoms or cycloalkyl having 3, 4, 5 or 6 C atoms;
R7 hydrogen or alkyl having 1, 2, 3 or 4 C atoms;
R8 and R9
independently of one another hydrogen, F, Cl, Br, OH, alkyl having 1, 2, 3 or 4 C atoms, CH₃O, CF₃, or CH₃SO₂;
R10 and 11
independently of one another R13-(CₘH₂ₘ)-Bₙ, where
m zero, 1, 2, 3 or 4;
n zero or 1;
B -CO-, -CONR14- or -SO₂-;
R14 hydrogen or alkyl having 1, 2, 3, 4, 5 or 6 C atoms;
R13 hydrogen, alkyl having 1, 2, 3 or 4 C atoms, cycloalkyl having 3, 4, 5 or 6 C atoms, 1-pyrrolidinyl, 1-piperidinyl, 1-(4-methylpiperazinyl), 1-morpholinyl, -COOR15, OR16, NR17R18 or phenyl which has independently of one another 1 or 2 substituents selected from the group of chlorine, fluorine, methyl and methoxy;
R15, R16, R17 and R18
independently of one another hydrogen or alkyl having 1, 2, 3, 4, 5 or 6 C atoms;
or
R10 and R11
form together with the nitrogen atom to which they are bonded a 4-, 5-, 6-, 7-, 8-, 9- or 10-membered ring in which one, two, three or four CH₂ groups may be replaced independently of one another by NR19, sulfur, oxygen, C(O) or SO₂;
R19 hydrogen, alkyl having 1, 2, 3 or 4 C atoms or cycloalkyl having 3, 4, 5 or 6 C atoms;
and the pharmaceutically acceptable salts and trifluoroacetates thereof.

2. A compound of the formula I as claimed in claim 1, in which the meanings are
R1, R2, R3 and R4
independently of one another hydrogen, F, Cl, Br, CN, CF₃, CH₃-SO₂, alkyl having 1, 2, 3 or 4 C atoms, NH₂, NH-CH₃ or N(CH₃)₂;
R5 and R6
independently of one another hydrogen, alkyl having 1, 2, 3, 4, 5 or 6 C atoms, CF₃-CH₂-, cycloalkyl having 3, 4, 5 or 6 C atoms or cyclopropyl-CH₂-,
or
R5 and R6
form together with the nitrogen atom to which they are bonded a 4-, 5-, 6-, 7-, 8-, 9- or 10-membered ring;
R7 hydrogen or methyl;
R8 and R9
independently of one another hydrogen, F, Cl, OH, alkyl having 1, 2, 3 or 4 C atoms, CH₃O, CF₃, or CH₃SO₂;
R10 and 11
independently of one another R13-(CₘH₂ₘ)-Bₙ, where
m zero, 1, 2, 3 or 4;
n zero or 1;
B -CO-, -CONR14- or -SO₂-;
R14 hydrogen or alkyl having 1, 2, 3, 4, 5 or 6 C atoms;
R13 hydrogen, alkyl having 1, 2, 3 or 4 C atoms, cycloalkyl having 3, 4, 5 or 6 C atoms, 1-pyrrolidinyl, 1-piperidinyl, 1-(4-methylpiperazinyl), 1-morpholinyl, -COOR15, OR16, NR17R18 or phenyl which has independently of one another 1 or 2 substituents selected from the group of chlorine, fluorine, methyl and methoxy; R15, R16, R17 and R18
independently of one another hydrogen or alkyl having 1, 2, 3, 4, 5 or 6 C atoms;
or
R10 and R11
form together with the nitrogen atom to which they are bonded a 4-, 5-, 6-, 7-, 8-, 9- or 10-membered ring in which one, two, three or four CH₂ groups may be replaced independently of one another by NR19, sulfur, oxygen, C(O) or SO₂;
R19 hydrogen or alkyl having 1, 2, 3 or 4 C atoms;
and the pharmaceutically acceptable salts and trifluoroacetates thereof.

3. A compound of the formula I as claimed in claim 1 and/or 2, in which the meanings are
R1, R2, R3 and R4 independently of one another hydrogen, F, Cl, Br, CN, CF₃, CH₃-SO₂,
methyl, ethyl, NH₂, NH-CH₃ or N(CH₃)₂;
R5 and R6
independently of one another hydrogen, methyl, ethyl, isopropyl, CF₃-CH₂ or cycloalkyl having 3, 4, 5 or 6 C atoms;
or
R5 and R6
form together with the nitrogen atom to which they are bonded a 4-, 5-, 6-, 7-, 8-, 9- or 10-membered ring;
R7 hydrogen or methyl;
R8 and R9
independently of one another hydrogen, Cl or methyl;
R10 and 11
independently of one another R13-(CₘH₂ₘ)-Bₙ, where
m zero, 1, 2, 3 or 4;
n zero or 1;
B -CO-, -CONR14- or -SO₂-;
R14 hydrogen or alkyl having 1, 2, 3, 4, 5 or 6 C atoms;
R13 hydrogen, methyl, ethyl, isopropyl, cycloalkyl having 3, 4, 5 or 6 C atoms, 1-pyrrolidinyl, 1-piperidinyl, 1-(4-methylpiperazinyl), -COOR15, OR16 or NR17R18;
R15, R16, R17 and R18
independently of one another hydrogen or alkyl having 1, 2, 3, 4, 5 or 6 C atoms;
or
R10 and R11
form together with the nitrogen atom to which they are bonded a 4-, 5-, 6-, 7-, 8-, 9- or 10-membered ring, in which one, two or three CH₂ groups may be replaced independently of one another by NR19, or C(O);
R19 hydrogen or methyl;
and the pharmaceutically acceptable salts and trifluoroacetates thereof.

4. A compound of the formula I as claimed in one or more of claims 1 to 3, in which the meanings are
R1 and R3
hydrogen;
R2 and R4
independently of one another hydrogen or Cl;
R5 and R6
independently of one another hydrogen or methyl;
or
R5 and R6
form together with the nitrogen atom to which they are bonded a 5- or 6-membered ring;
R7 hydrogen;
R8 and R9
independently of one another hydrogen or Cl;
R10 and 11
independently of one another R13-(CₘH₂ₘ)-Bₙ, where
m zero, 1, 2, 3 or 4;
n zero or 1;
B -CO- or -CONR14-;
R14 hydrogen or methyl;
R13hydrogen, methyl, cycloalkyl having 3, 4, 5 or 6 C atoms, 1-pyrrolidinyl, 1-piperidinyl, 1-(4-methylpiperazinyl), -COOR15, OR16 or NR17R18;
R15, R16, R17 and R18
independently of one another hydrogen or alkyl having 1, 2, 3, 4, 5 or 6 C atoms;
or
R10 and R11
form together with the nitrogen atom to which they are bonded a 4-, 5-, 6-, 7-, 8-, 9- or 10-membered ring in which one, two or three CH₂ groups may be replaced independently of one another by NR19, or C(O) ;
R19 hydrogen or methyl;
and the pharmaceutically acceptable salts and trifluoroacetates thereof.

5. A compound of the formula I as claimed in one or more of claims 1 to 4 selected from the group
1-amino-4-(4-aminophenyl)-6-chloro-3,4-dihydroisoquinoline,
1-amino-4-(4-aminophenyl)- 3,4-dihydroisoquinoline,
1-amino-4-(2-aminophenyl)-6-chloro-3,4-dihydroisoquinoline,
4-(4-aminophenyl)-6-chloro-1-methylamino-3,4-dihydroisoquinoline,
4-(4-aminophenyl)-6-chloro-1-dimethylamino-3,4-dihydroisoquinoline,
4-(4-aminophenyl)-6-chloro-1-(N-pyrrolidino)-3,4-dihydroisoquinoline,
N-(ethoxycarbonymethyl)-N'-4-[(6-chloro-3,4-dihydro-1-dimethylaminoisoquinoline-4-yl)phenyl]urea and
3-{4-[(6-chloro-3,4-dihydro-1-dimethylaminoisoquinolin-4-yl)phenyl]}imidazolidine-2,4-dione
and the pharmaceutically acceptable salts and trifluoroacetates thereof.

6. A compound of the formula I and the pharmaceutically acceptable salts thereof as claimed in one or more of claims 1 to 5 for use as a medicament.

7. The use of a compound of the formula I and/or its pharmaceutically acceptable salts as claimed in one or more of claims 1 to 5 for the manufacture of a medicament for the treatment or prophylaxis of impairments of respiratory drive, of respiratory disorders, sleep-related respiratory disorders, sleep apneas, of snoring, of acute and chronic renal disorders, of acute renal failure and of chronic renal failure, of impairments of bowel function, of high blood pressure, of essential hypertension, of central nervous system disorders, of disorders resulting from CNS overexcitability, epilepsy and centrally induced spasms or of anxiety states, depressions and psychoses, of ischemic states of the peripheral or central nervous system or of stroke, degenerative CNS disorders, reduced memory capacity, dementia and Alzheimer's disease, and of acute and chronic damage and disorders of peripheral organs or limbs caused by ischemic or reperfusion events, of atherosclerosis, of impairments of lipid metabolism, of thromboses, of impairments of biliary function, of infestation by ectoparasites, of disorders resulting from endothelial dysfunction, of protozoal diseases, of malaria, of states of shock or of diabetes and late damage from diabetes or of diseases in which cell proliferation represents a primary or secondary cause, for the preservation and storage of transplants for surgical procedures, for use in surgical operations and organ transplantations and for maintaining health and prolonging life.

8. The use of a compound of the formula I and/or its pharmaceutically acceptable salts as claimed in one or more of claims 1 to 5 in combination with other pharmaceuticals or active ingredients for the manufacture of a medicament for the treatment or prophylaxis of impairments of respiratory drive, of respiratory disorders, sleep-related respiratory disorders, sleep apneas, of snoring, of acute and chronic renal disorders, of acute renal failure and of chronic renal failure, of impairments of bowel function, of high blood pressure, of essential hypertension, of central nervous system disorders, of disorders resulting from CNS overexcitability, epilepsy and centrally induced spasms or of anxiety states, depressions and psychoses, of ischemic states of the peripheral or central nervous system or of stroke, of acute and chronic damage and disorders of peripheral organs or limbs caused by ischemic or reperfusion events, of atherosclerosis, of impairments of lipid metabolism, of thromboses, of impairments of biliary function, of infestation by ectoparasites, of disorders resulting from endothelial dysfunction, of protozoal diseases, of malaria, of states of shock or of diabetes and late damage from diabetes or of diseases in which cell proliferation represents a primary or secondary cause, for the preservation and storage of transplants for surgical procedures, for use in surgical operations and organ transplantations and for maintaining health and prolonging life.

9. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of impairments of the respiratory drive and/or of sleep-related respiratory impairments such as sleep apneas.

10. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of snoring.

11. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of acute or chronic renal disorders, of acute renal failure or of chronic renal failure.

12. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of impairments of bowel function.

13. A pharmaceutical preparation for human, veterinary or phytoprotective use comprising an effective amount of a compound of the formula I and/or of a pharmaceutically acceptable salt thereof as claimed in one or more of claims 1 to 5.

14. A pharmaceutical preparation for human, veterinary or phytoprotective use comprising an effective amount of a compound of the formula I and/or of a pharmaceutically acceptable salt thereof as claimed in one or more of claims 1 to 5, in combination with other pharmacological active ingredients or pharmaceuticals.

## Revendications

1. Composés de formule I 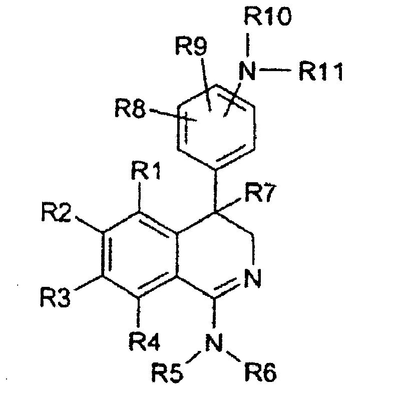 dans laquelle
R1, R2, R3 et R4 signifient, indépendamment l'un de l'autre, hydrogène, F, Cl, Br, I, CN, NO₂, CF₃, CH₃-SO₂, alkyle comprenant 1, 2, 3 ou 4 atomes de carbone, NH₂, NH-CH₃ ou N(CH₃)₂ ;
R5 et R6 signifient, indépendamment l'un de l'autre hydrogène, alkyle comprenant 1, 2, 3, 4, 5 ou 6 atomes de carbone, CF₃-CH₂-, cycloalkyle comprenant 3, 4, 5 ou 6 atomes de carbone ou cyclopropyl-CH₂-, ou
R5 et R6 forment ensemble avec l'atome d'azote auquel ils sont liés, un cycle de 4, 5, 6, 7, 8, 9 ou 10 chaînons, dans lequel un ou deux groupes CH₂ peuvent être remplacés, indépendamment l'un de l'autre, par NR12, soufre, oxygène, C(O) ou SO₂ ;
R12 signifie hydrogène, alkyle comprenant 1, 2, 3 ou 4 atomes de carbone ou cycloalkyle comprenant 3, 4, 5 ou 6 atomes de carbone ;
R7 signifie hydrogène ou alkyle comprenant 1, 2, 3 ou 4 atomes de carbone ;
R8 et R9 signifient, indépendamment l'un de l'autre, hydrogène, F, Cl, Br, OH, alkyle comprenant 1, 2, 3 ou 4 atomes de carbone, CH₃O, CF₃, ou CH₃SO₂ ;
R10 et R11 signifient, indépendamment l'un de l'autre, R13-(CₘH₂ₘ)-Bₙ, où
m vaut zéro, 1, 2, 3 ou 4 ;
n vaut zéro ou 1 ;
B signifie -CO-, -CONR14- ou -SO₂- ;
R14 signifie hydrogène ou alkyle comprenant 1, 2, 3, 4, 5 ou 6 atomes de carbone ;
R13 signifie hydrogène, alkyle comprenant 1, 2, 3 ou 4 atomes de carbone, cycloalkyle comprenant 3, 4, 5 ou 6 atomes de carbone, 1-pyrrolidinyle, 1-pipéridinyle, 1-(4-méthylpipérazinyle), 1-morpholinyle, -COOR15, OR16, NR17R18 ou phényle, qui porte, indépendamment l'un de l'autre, 1 ou 2 substituants, choisis dans le groupe chlore, fluor, méthyle et méthoxy ;
R15, R16, R17 et R18 signifient, indépendamment l'un de l'autre, hydrogène ou alkyle comprenant 1, 2, 3, 4, 5 ou 6 atomes de carbone ;
ou
R10 et R11 forment ensemble avec l'atome d'azote auquel ils sont liés, un cycle de 4, 5, 6, 7, 8, 9 ou 10 chaînons, dans lequel un, deux, trois ou quatre groupes CH₂ peuvent être remplacés, indépendamment l'un de l'autre, par NR19, soufre, oxygène, C(O) ou SO₂ ;
R19 signifie hydrogène, alkyle comprenant 1, 2, 3 ou 4 atomes de carbone ou cycloalkyle comprenant 3, 4, 5 ou 6 atomes de carbone ;
ainsi que leurs sels pharmaceutiquement acceptables et leurs trifluoroacétates.

2. Composés de formule I selon la revendication 1,
où :
R1, R2, R3 et R4 signifient, indépendamment l'un de l'autre, hydrogène, F, Cl, Br, CN, CF₃, CH₃-SO₂, alkyle comprenant 1, 2, 3 ou 4 atomes de carbone, NH₂, NH-CH₃ ou N(CH₃)₂ ;
R5 et R6 signifient, indépendamment l'un de l'autre hydrogène, alkyle comprenant 1, 2, 3, 4, 5 ou 6 atomes de carbone, CF₃-CH₂-, cycloalkyle comprenant 3, 4, 5 ou 6 atomes de carbone ou cyclopropyl-CH₂-,
ou
R5 et R6 forment ensemble, avec l'atome d'azote auquel ils sont liés, un cycle de 4, 5, 6, 7, 8, 9 ou 10 chaînons ;
R7 signifie hydrogène ou méthyle ;
R8 et R9 signifient, indépendamment l'un de l'autre, hydrogène, F, Cl, OH, alkyle comprenant 1, 2, 3 ou 4 atomes de carbone, CH₃O, CF₃, ou CH₃SO₂ ;
R10 et R11 signifient, indépendamment l'un de l'autre, R13-(CₘH₂ₘ)-Bₙ, où
m vaut zéro, 1, 2, 3 ou 4 ;
n vaut zéro ou 1 ;
B signifie -CO-, -CONR14- ou -SO₂- ;
R14 signifie hydrogène ou alkyle comprenant 1, 2, 3, 4, 5 ou 6 atomes de carbone ;
R13 signifie hydrogène, alkyle comprenant 1, 2, 3 ou 4 atomes de carbone, cycloalkyle comprenant 3, 4, 5 ou 6 atomes de carbone, 1-pyrrolidinyle, 1-pipéridinyle, 1-(4-méthylpipérazinyle), 1-morpholinyle, -COOR15, OR16, NR17R18 ou phényle, qui porte, indépendamment l'un de l'autre, 1 ou 2 substituants, choisis dans le groupe chlore, fluor, méthyle et méthoxy ;
R15, R16, R17 et R18 signifient, indépendamment l'un de l'autre, hydrogène ou alkyle comprenant 1, 2, 3, 4, 5 ou 6 atomes de carbone ;
ou
R10 et R11 forment ensemble avec l'atome d'azote auquel ils sont liés, un cycle de 4, 5, 6, 7, 8, 9 ou 10 chaînons, dans lequel un, deux, trois ou quatre groupes CH₂ peuvent être remplacés, indépendamment l'un de l'autre, par NR19, soufre, oxygène, C(O) ou SO₂ ;
R19 signifie hydrogène ou alkyle comprenant 1, 2, 3 ou 4 atomes de carbone ;
ainsi que leurs sels pharmaceutiquement acceptables et leurs trifluoroacétates.

3. Composés de formule I selon la revendication 1 et/ou 2, où
R1, R2, R3 et R4 signifient, indépendamment l'un de l'autre, hydrogène, F, Cl, Br, CN, CF₃, CH₃-SO₂, méthyle, éthyle, NH₂, NH-CH₃ ou N(CH₃)₂ ;
R5 et R6 signifient, indépendamment l'un de l'autre, hydrogène, méthyle, éthyle, isopropyle, CF₃-CH₂ ou cycloalkyle comprenant 3, 4, 5 ou 6 atomes de carbone ;
ou
R5 et R6 forment ensemble, avec l'atome d'azote auquel ils sont liés, un cycle de 4, 5, 6, 7, 8, 9 ou 10 chaînons ;
R7 signifie hydrogène ou méthyle ;
R8 et R9 représentent, indépendamment l'un de l'autre hydrogène, C1 ou méthyle ;
R10 et R11 signifient, indépendamment l'un de l'autre, R13-(CₘH₂ₘ)-Bₙ, où
m vaut zéro, 1, 2, 3 ou 4 ;
n vaut zéro ou 1 ;
B signifie -CO-, -CONR14- ou -SO₂- ;
R14 signifie hydrogène ou alkyle comprenant 1, 2, 3, 4, 5 ou 6 atomes de carbone ;
R13 signifie hydrogène, méthyle, éthyle, isopropyle, cycloalkyle comprenant 3, 4, 5 ou 6 atomes de carbone, 1-pyrrolidinyle, 1-pipéridinyle, 1-(4-méthylpipérazinyle), -COOR15, OR16 ou NR17R18 ;
R15, R16, R17 et R18 signifient, indépendamment l'un de l'autre, hydrogène ou alkyle comprenant 1, 2, 3, 4, 5 ou 6 atomes de carbone ;
ou
R10 et R11 forment ensemble avec l'atome d'azote auquel ils sont liés, un cycle de 4, 5, 6, 7, 8, 9 ou 10 chaînons, dans lequel un, deux ou trois groupes CH₂ peuvent être remplacés, indépendamment l'un de l'autre, par NR19, ou C(O) ; R19 signifie hydrogène ou méthyle ;
ainsi que leurs sels pharmaceutiquement acceptables et leurs trifluoroacétates.

4. Composés de formule I selon l'une ou plusieurs des revendications 1 à 3, où
R1 et R3 signifient hydrogène ;
R2 et R4 signifient, indépendamment l'un de l'autre hydrogène ou Cl ;
R5 et R6 signifient, indépendamment l'un de l'autre hydrogène ou méthyle ;
ou
R5 et R6 forment ensemble avec l'atome de N auquel ils sont liés un cycle de 5 ou 6 chaînons ;
R7 signifie hydrogène ;
R8 et R9 signifient, indépendamment l'un de l'autre hydrogène ou Cl ;
R10 et R11 signifient, indépendamment l'un de l'autre, R13-(CₘH₂ₘ)-Bₙ, où
m vaut zéro, 1, 2, 3 ou 4 ;
n vaut zéro ou 1 ;
B signifie -CO- ou -CONR14- ;
R14 signifie hydrogène ou méthyle ;
R13 signifie hydrogène, méthyle, cycloalkyle comprenant 3, 4, 5 ou 6 atomes de carbone, 1-pyrrolidinyle, 1-pipéridinyle, 1-(4-méthylpipérazinyle), -COOR15, OR16 ou NR17R18 ; R15, R16, R17 et R18 signifient, indépendamment
l'un de l'autre, hydrogène ou alkyle comprenant 1, 2, 3, 4, 5 ou 6 atomes de carbone ;
ou
R10 et R11 forment ensemble avec l'atome d'azote auquel ils sont liés, un cycle de 4, 5, 6, 7, 8, 9 ou 10 chaînons, dans lequel un, deux, trois ou quatre groupes CH₂ peuvent être remplacés, indépendamment l'un de l'autre, par NR19, ou C(O) ;
R19 signifie hydrogène ou méthyle ;
ainsi que leurs sels pharmaceutiquement acceptables et leurs trifluoroacétates.

5. Composés de formule I selon l'une ou plusieurs des revendications 1 à 4, choisis dans le groupe formé par
- la 1-amino-4-(4-aminophényl)-6-chloro-3,4-dihydro-isoquinoléine,
- la 1-amino-4-(4-aminophényl)-3,4-dihydro-isoquinoléine,
- la 1-amino-4-(2-aminophényl)-6-chloro-3,4-dihydro-isoquinoléine,
- la 4-(4-aminophényl)-6-chloro-1-méthylamino-3,4-dihydro-isoquinoléine,
- la 4-(4-aminophényl)-6-chloro-1-diméthylamino-3,4-dihydro-isoquinoléine,
- la 4-(4-aminophényl)-6-chloro-1-(N-pyrrolidino)-3,4-dihydro-isoquinoléine,
- la N-(éthoxycarbonylméthyl)-N'-4-[(6-chloro-3,4-dihydro-1-diméthylamino-isoquinoléin-4-yl)phényl]urée
et
- la 3-{4-[(6-chloro-3,4-dihydro-1-diméthylamino-isoquinoléin-4-yl)phényl]}-imidazolidine-2,4-dione
ainsi que leurs sels pharmaceutiquement acceptables et leurs trifluoroacétates.

6. Composés de formule I et leurs sels pharmaceutiquement acceptables selon l'une ou plusieurs des revendications 1 à 5 pour une utilisation comme médicament.

7. Utilisation d'un composé de formule I et/ou de ses sels pharmaceutiquement acceptables selon l'une ou plusieurs des revendications 1 à 5 pour la préparation d'un médicament destiné au traitement ou à la prophylaxie de troubles de l'effort respiratoire, de troubles respiratoires, de troubles respiratoires provoqués par le sommeil, des apnées du sommeil, du ronflement, de maladies aiguës et chroniques des reins, de l'insuffisance rénale aiguë et de l'insuffisance rénale chronique, de troubles de la fonction intestinale, de l'hypertension, de l'hypertonie essentielle, de maladies du système nerveux central, de maladies qui résultent d'une surexcitabilité du SNC, de l'épilepsie et de crampes déclenchées de manière centrale ou d'états d'anxiété, de dépressions et de psychoses, d'états ischémiques du système nerveux périphérique ou central ou d'une attaque, de maladies de dégénérescence du SNC, de la diminution de la puissance de la mémoire, de la démence et de la maladie d'Alzheimer, de dommages et de maladies aigu(ë)s et chroniques d'organes périphériques ou de membres, qui sont provoqué(e)s par des événements d'ischémie ou de reperfusion, de l'athérosclérose, de troubles du métabolisme des graisses, de thromboses, de troubles de la fonction biliaire, d'une attaque par des ectoparasites, de maladies suite à un dysfonctionnement endothélial, de maladies dues à des protozoaires, de la malaria, au traitement ou à la prophylaxie d'états de choc ou du diabète et des dommages diabétiques tardifs
ou de maladies dans lesquelles la prolifération cellulaire représente une cause primaire ou secondaire, à la conservation et à l'entreposage de greffes destinées à des interventions chirurgicales, à une utilisation lors d'opérations chirurgicales et de transplantations d'organes et aux soins de santé et à la prolongation de la vie.

8. Utilisation d'un composé de formule I et/ou de ses sels pharmaceutiquement acceptables selon l'une ou plusieurs des revendications 1 à 5 en combinaison avec d'autres médicaments ou substances actives pour la préparation d'un médicament destiné au traitement ou à la prophylaxie de troubles de l'effort respiratoire, de troubles respiratoires, de troubles respiratoires provoqués par le sommeil, des apnées du sommeil, du ronflement, de maladies aiguës et chroniques des reins, de l'insuffisance rénale aiguë et de l'insuffisance rénale chronique, de troubles de la fonction intestinale, de l'hypertension, de l'hypertonie essentielle, de maladies du système nerveux central, de maladies qui résultent d'une surexcitabilité du SNC, de l'épilepsie et de crampes déclenchées de manière centrale ou d'états d'anxiété, de dépressions et de psychoses, d'états ischémiques du système nerveux périphérique ou central ou d'une attaque, de dommages et de maladies aigu(ë)s et chroniques d'organes périphériques ou de membres, qui sont provoqué(e)s par des événements d'ischémie ou de reperfusion, de l'athérosclérose, de troubles du métabolisme des graisses, de thromboses, de troubles de la fonction biliaire, d'une attaque par des ectoparasites, de maladies suite à un dysfonctionnement endothélial, de maladies dues à des protozoaires, de la malaria, au traitement ou à la prophylaxie d'états de choc ou du diabète et des dommages diabétiques tardifs ou de maladies dans lesquelles la prolifération cellulaire représente une cause primaire ou secondaire, à la conservation et à l'entreposage de greffes destinées à des interventions chirurgicales, à une utilisation lors d'opérations chirurgicales et de transplantations d'organes et aux soins de santé et à la prolongation de la vie.

9. Utilisation d'un composé de formule I et/ou de ses sels pharmaceutiquement acceptables selon l'une ou plusieurs des revendications 1 à 5, seul (s) ou en combinaison avec d'autres médicaments ou substances actives pour la préparation d'un médicament destiné au traitement ou à la prophylaxie de troubles de l'effort respiratoire et/ou de troubles respiratoires provoqués par le sommeil, tels que les apnées du sommeil.

10. Utilisation d'un composé de formule I et/ou de ses sels pharmaceutiquement acceptables selon l'une ou plusieurs des revendications 1 à 5, seul(s) ou en combinaison avec d'autres médicaments ou substances actives pour la préparation d'un médicament destiné au traitement ou à la prophylaxie du ronflement.

11. Utilisation d'un composé de formule I et/ou de ses sels pharmaceutiquement acceptables selon l'une ou plusieurs des revendications 1 à 5, seul(s) ou en combinaison avec d'autres médicaments ou substances actives pour la préparation d'un médicament destiné au traitement ou à la prophylaxie de maladies rénales aiguës ou chroniques, de l'insuffisance rénale aiguë ou de l'insuffisance rénale chronique.

12. Utilisation d'un composé de formule I et/ou de ses sels pharmaceutiquement acceptables selon l'une ou plusieurs des revendications 1 à 5, seul(s) ou en combinaison avec d'autres médicaments ou substances actives pour la préparation d'un médicament destiné au traitement ou à la prophylaxie de troubles de la fonction intestinale.

13. Préparation pharmaceutique destinée à une utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 5.

14. Préparation pharmaceutique destinée à une utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 5, en combinaison avec d'autres substances actives pharmacologiques ou d'autres médicaments.
